# EUROPEAN PATENT SPECIFICATION

(11) **EP 2 744 053 B1**
(45) Date of publication and mention of the grant of the patent: **15.05.2019**
(21) Application number: 12820110.0
(22) Date of filing: 26.07.2012
(51) Int. Cl.: H01S 3/106, A61B 8/00, G01N 29/00, H01S 3/11, A61B 5/00, G01N 29/24, G01N 29/11, G01N 29/46, G01N 21/17, A61B 5/1455, H01S 3/092, H01S 3/16, A61B 5/145, H01S 3/08, H01S 3/06, H01S 3/102

(54) **LASER LIGHT SOURCE UNIT, CONTROL METHOD FOR SAME, AND DEVICE AND METHOD FOR GENERATING PHOTOACOUSTIC IMAGE**
LASERLICHTQUELLENEINHEIT, STEUERVERFAHREN DAFÜR SOWIE VORRICHTUNG UND VERFAHREN ZUR ERZEUGUNG EINES PHOTOAKUSTISCHEN BILDES
UNITÉ SOURCE DE LUMIÈRE LASER, SON PROCÉDÉ DE COMMANDE, ET DISPOSITIF ET PROCÉDÉ DE GÉNÉRATION D'IMAGE PHOTO-ACOUSTIQUE

(30) Priority: 29.07.2011 JP 2011166980; 19.07.2012 JP 2012160428
(43) Date of publication of application: 18.06.2014
(73) Proprietor: FUJIFILM Corporation, Tokyo 106-8620 (JP)
(72) Inventor: IRISAWA Kaku, Ashigarakami-gun Kanagawa 258-8538 (JP); KASAMATSU Tadashi, Ashigarakami-gun Kanagawa 258-8538 (JP)
(74) Representative: Klunker IP Patentanwälte PartG mbB
(86) International application number: PCT/JP2012/068915
(87) International publication number: WO 2013/018634

(56) References cited:
- WO-A1-2012/093654
- WO-A2-2011/022547
- JP-A- 9 326 521
- JP-A- 2000 105 464
- JP-A- 2001 044 548
- JP-A- 2006 324 601
- JP-A- 2007 081 065
- JP-A- 2011 083 531
- US-A- 3 609 586
- US-A- 4 891 738
- US-A- 5 107 509
- US-A1- 2006 039 436
- US-A1- 2009 237 661
- US-A1- 2011 150 015
- XUEDING WANG ET AL: "A high-speed photoacoustic tomography system based on a commercial ultrasound and a custom transducer array", PROCEEDINGS OF SPIE, vol. 7564, 11 February 2010 (2010-02-11), pages 756424-756424-9, XP055164311, ISSN: 0277-786X, DOI: 10.1117/12.842666

## Description

### BACKGROUND OF THE INVENTION

### 1. Field of the Invention

The present invention relates to a laser source unit and a control method thereof, and more particularly, to a laser source unit capable of switching and emitting a laser beam having a plurality of wavelengths, and a control method thereof.

In addition, the present invention relates to a photoacoustic image generation apparatus and a photoacoustic image generation method, and more particularly, to a photoacoustic image generation apparatus which irradiates an object with a laser beam having a plurality of wavelengths to detect a photoacoustic signal and generates a photoacoustic image on the basis of the detected photoacoustic signal, and a photoacoustic image generation method.

### 2. Description of the Related Art

Hitherto, for example, as disclosed in JP2005-21380A and A High-Speed Photoacoustic Tomography System based on a Commercial Ultrasound and a Custom Transducer Array, Xueding Wang, Jonathan Cannata, Derek DeBusschere, Changhong Hu, J. Brian Fowlkes, and Paul Carson, Proc. SPIE Vol. 7564, 756424 (Feb.23, 2010), a photoacoustic image forming apparatus that forms an image of the inside of a living body using a photoacoustic effect has been known. In the photoacoustic image forming apparatus, a living body is irradiated with pulsed light such as a pulse laser beam. Body tissues absorbing energy of the pulsed light expand in volume inside the living body irradiated with the pulsed light, and thus acoustic waves are generated. It is possible to detect the acoustic waves using an ultrasonic probe or the like, and to form a visible image of the inside of the living body on the basis of the detected signal (photoacoustic signal). In a photoacoustic image forming method, acoustic waves are generated in a specific light absorber, and thus it is possible to form an image of specific tissues in the living body, for example, blood vessels.

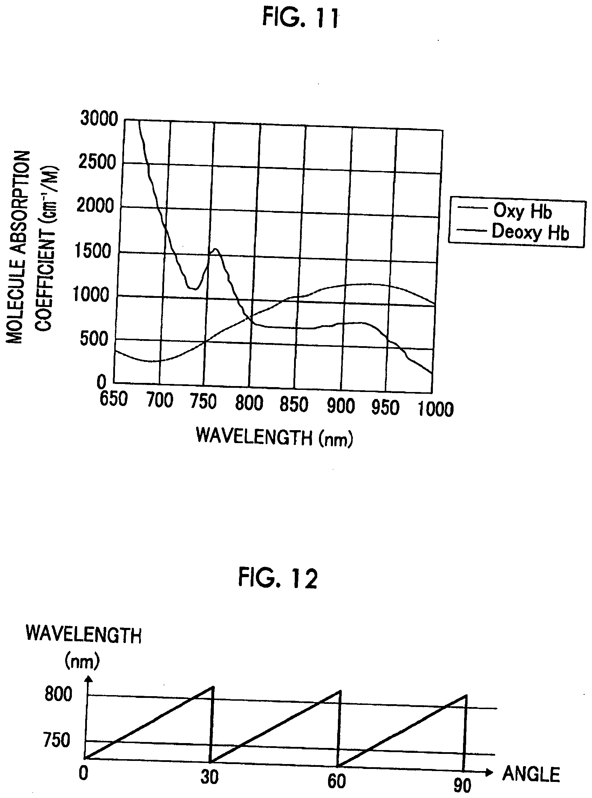

Incidentally, many of body tissues have an optical absorption property varying depending on a wavelength of light, and generally, the optical absorption property is unique for each tissue. For example, Fig. 11 illustrates molecular absorption coefficients of oxygenated hemoglobin (hemoglobin combined with oxygen: oxy-Hb) which is contained in a large amount in an artery of a human and deoxygenated hemoglobin (hemoglobin not combined with oxygen: deoxy-Hb) which is contained in a large amount in a vein, according to light wavelengths. An optical absorption property of an artery corresponds to that of oxygenated hemoglobin, and an optical absorption property of a vein corresponds to that of deoxygenated hemoglobin. There is known a photoacoustic image forming method of irradiating blood vessel parts with a light beam having two different types of wavelengths and of distinctively forming images of an artery and a vein (for example, see JP2010-046215A), using a difference in light absorptivity according to the wavelengths.

Herein, with regard to a variable wavelength laser, JP2009-231483A discloses that a laser beam having a desired wavelength is obtained by disposing an etalon or a birefringent filter as a wavelength selection element within an optical resonator and adjusting the rotation angle thereof. In addition, JP2000-105464A discloses that an etalon as wavelength selection means is disposed within an optical resonator and that the etalon is scanned at a constant speed. JP2000-105464A discloses that laser oscillation is performed only when a transmission wavelength of the etalon is consistent with longitudinal mode oscillation of a laser beam and that the oscillation of the laser beam is performed in a pulsed manner when a scanning speed of the etalon is increased.

### SUMMARY OF THE INVENTION

It is considered that when a laser beam having a plurality of wavelengths is switched and emitted using a birefringent filter, the birefringent filter is rotated at a predetermined rotation speed and the wavelengths are sequentially switched. For example, when a pulse laser beam having a wavelength of 750 nm and a pulse laser beam having a wavelength of 800 nm are switched and emitted, a Q switch inserted into an optical resonator is controlled at a timing when the birefringent filter is set to be at a rotational displacement position corresponding to the wavelength of 750 nm and at a timing when the birefringent filter is set to be at a rotational displacement position corresponding to the wavelength of 800 nm, and thus pulse laser beams having two wavelengths (750 nm and 800 nm) can be irradiated.

Fig. 12 illustrates change characteristics of a transmission wavelength (oscillation wavelength of optical resonator) for a rotational displacement of a birefringent filter. The birefringent filter changes the transmission wavelength between 740 nm and 810 nm every time rotational displacement by 30 degrees occurs, and repeats a free spectral range (FSR) twelve times for one rotation. When the birefringent filter is rotated once per second, the birefringent filter repeats the FSR at a frequency of 12 Hz.

When the birefringent filter having change characteristics of a transmission wavelength illustrated in Fig. 12 is rotated once per second, the birefringent filter repeats the FSR at a frequency of 12 Hz, and thus a period with which a pulse laser beam having each wavelength is irradiated is set to 83 ms (1/12 seconds). For example, a time interval between the emission of the pulse laser beam having a wavelength of 750 nm and the emission of the next pulse laser beam having a wavelength of 750 nm is set to 83 ms. In this manner, a time interval of emission between pulse laser beams having the same wavelength depends on a repetition period of the FSR. Thus, it is not possible to shorten a period at which a pulse laser beam having each wavelength is irradiated as long as a rotation speed of the birefringent filter is not increased.

The invention is contrived in view of such situations, and an object thereof is to provide a laser source unit capable of switching and emitting a plurality of wavelengths at a high speed without increasing a rotation speed (angular velocity) of a birefringent filter, and a control method thereof. In addition, the invention provides a photoacoustic image generation apparatus including the laser source unit and a photoacoustic image generation method using the control method of the laser source unit.

The invention is defined in claim 1. In order to achieve the above-described object, the invention provides a laser source unit that emits pulse laser beams having a plurality of different wavelengths, the laser source unit including: a laser rod; an excitation light source that irradiates the laser rod with excitation light; an optical resonator that includes a pair of mirrors facing each other with the laser rod interposed therebetween; a Q switch which is inserted into the optical resonator; a birefringent filter which is inserted into the optical resonator and changes an oscillation wavelength of the optical resonator in association with rotational displacement of the birefringent filter; driving means that reciprocatively rotates the birefringent filter in a predetermined range including a position at which change characteristics of the oscillation wavelength for the rotational displacement of the birefringent filter are set to be a discontinuous point; and an emission control unit that irradiates the laser rod with excitation light from the excitation light source and then turns on the Q switch, at a timing when a rotational displacement position of the birefringent filter is set to a position corresponding to the wavelength of the pulse laser beam to be emitted, to cause the pulse laser beam to be emitted.

In the invention, the driving means may include a rotating body that circularly moves and a rod that connects the rotating body and the birefringent filter, and may have a crank mechanism converting a circular motion of the rotating body to a reciprocating rotational motion of the birefringent filter.

The rotating body may be rotated at a fixed speed.

The plurality of wavelengths may include first and second wavelengths. The oscillation wavelength of the optical resonator may be set to the first wavelength at a first rotational displacement position to which the birefringent filter is rotated in a first rotation direction by a first amount from the discontinuous point, and the oscillation wavelength of the optical resonator may be set to the second wavelength at a second rotational displacement position to which the birefringent filter is rotated in a second direction, opposite to the first direction, by a second amount from the discontinuous point.

The birefringent filter may repeat a free spectral range including the first wavelength and the second wavelength a plurality of times during one rotation in a fixed direction.

The predetermined range in which the birefringent filter is reciprocatively rotated may be smaller than a range of the rotational displacement of the birefringent filter which corresponds to one free spectral range.

The oscillation wavelength of the optical resonator may change to a sawtooth wave pattern for the rotational displacement of the birefringent filter.

The invention also provides a photoacoustic image generation apparatus including: a laser source unit that emits pulse laser beams having a plurality of different wavelengths; sampling means that samples a photoacoustic signal, generated within an object when the object is irradiated with the pulse laser beam having a plurality of wavelengths, to generate pieces of photoacoustic data corresponding to the respective wavelengths; phase information extraction means that generates phase information indicating a magnitude relation between relative signal intensities of the pieces of photoacoustic data corresponding to the respective wavelengths; and photoacoustic image construction means that generates a photoacoustic image on the basis of the phase information. The laser source unit includes a laser rod, an excitation light source that irradiates the laser rod with excitation light, an optical resonator that includes a pair of mirrors facing each other with the laser rod interposed therebetween, a Q switch which is inserted into the optical resonator, a birefringent filter which is inserted into the optical resonator and changes an oscillation wavelength of the optical resonator in association with rotational displacement of the birefringent filter, driving means that reciprocatively rotates the birefringent filter in a predetermined range including a position at which change characteristics of the oscillation wavelength for the rotational displacement of the birefringent filter are set to be a discontinuous point, and an emission control unit that irradiates the laser rod with excitation light from the excitation light source and then turns on the Q switch, at a timing when a rotational displacement position of the birefringent filter is set to a position corresponding to the wavelength of the pulse laser beam to be emitted, to cause the pulse laser beam to be emitted.

The photoacoustic image generation apparatus may further include intensity information extraction means that generates intensity information indicating signal intensity on the basis of the pieces of photoacoustic data corresponding to the respective wavelengths. The photoacoustic image construction means may determine a gradation value of each pixel of the photoacoustic image on the basis of the intensity information and may determine a display color of each pixel on the basis of the phase information.

The plurality of wavelengths may include first and second wavelengths. The photoacoustic image generation apparatus may further include: complex number creation means that generates complex number data in which any one of first photoacoustic data corresponding to a photoacoustic signal, detected when irradiation with a pulse laser beam having the first wavelength is performed, and second photoacoustic data corresponding to a photoacoustic signal, detected when irradiation with a pulse laser beam having the second wavelength is performed, is set to a real part and the other one is set to an imaginary part; and photoacoustic image reconstruction means that generates a reconstructed image from the complex number data using a Fourier transform method. The phase information extraction means may extract the phase information from the reconstructed image, and the intensity information extraction means may extract the intensity information from the reconstructed image.

The sampling means may further sample reflected acoustic waves with respect to acoustic waves transmitted to the object to generate reflected acoustic wave data. The photoacoustic image generation apparatus may further include acoustic wave image generation means that generates an acoustic wave image on the basis of the reflected acoustic wave data.

The invention further provides a control method of a laser source unit that emits pulse laser beams having a plurality of different wavelengths, the control method including: a step of reciprocatively rotating, in a predetermined range, a birefringent filter which is inserted into an optical resonator including a pair of mirrors facing each other with a laser rod interposed therebetween and changes an oscillation wavelength of the optical resonator in association with rotational displacement of the birefringent filter; and a step of irradiating the laser rod with excitation light, and after the irradiation with the excitation light, turning on a Q switch inserted into the optical resonator, at a timing when a rotational displacement position of the birefringent filter is set to a position corresponding to the wavelength of the pulse laser beam to be emitted, to cause the pulse laser beam to be emitted.

The invention also provides a photoacoustic image generation method including: a step, which is a step of emitting pulse laser beams having a plurality of different wavelengths, of irradiating a laser rod with excitation light while reciprocatively rotating a birefringent filter, which is inserted into an optical resonator including a pair of mirrors facing each other with the laser rod interposed therebetween and changes an oscillation wavelength of the optical resonator in association with rotational displacement of the birefringent filter, in a predetermined range, and after the irradiation with the excitation light, turning on a Q switch inserted into the optical resonator, at a timing when a rotational displacement position of the birefringent filter is set to a position corresponding to the wavelength of the pulse laser beam to be emitted, to cause the pulse laser beam to be emitted; a step of sampling a photoacoustic signal generated within an object when the object is irradiated with the pulse laser beam having a plurality of wavelengths to generate pieces of photoacoustic data corresponding to the respective wavelengths; a step of generating phase information indicating a magnitude relation between relative signal intensities of the pieces of photoacoustic data corresponding to the respective wavelengths, and a step of generating a photoacoustic image on the basis of the phase information.

In a photoacoustic image generation apparatus and an acoustic wave unit of the invention, a birefringent filter inserted into an optical resonator and changing an oscillation wavelength in association with rotational displacement of the birefringent filter is reciprocatively rotated in a predetermined range including a discontinuous point at which change characteristics of the oscillation wavelength for the rotational displacement are discontinuous, and a Q switch inserted into the optical resonator is turned on at a timing when a rotational displacement position of the birefringent filter is set to a position corresponding to a wavelength of a pulse laser beam to be emitted. At a discontinuous point of change characteristics of a transmission wavelength for the rotational displacement of the birefringent filter, a change in transmission wavelength for the rotational displacement becomes greater. By using this feature, it is possible to switch and emit a plurality of wavelengths at a high speed without increasing a rotation speed (angular velocity) of the birefringent filter.

### BRIEF DESCRIPTION OF THE DRAWINGS

Fig. 1 is a block diagram of a photoacoustic image generation apparatus according to a first embodiment of the invention.
Fig. 2 is a block diagram illustrating a configuration of a laser source unit according to the first embodiment.
Fig. 3 is a diagram illustrating a configuration example of driving means that reciprocatively rotates a birefringent filter.
Fig. 4 is a graph illustrating change characteristics of a transmission wavelength for rotational displacement of the birefringent filter.
Fig. 5 is a timing chart illustrating various types of triggers and an emission timing.
Fig. 6 is a flow chart illustrating an operation procedure of the photoacoustic image generation apparatus according to the first embodiment.
Fig. 7 is a timing chart illustrating various types of triggers and an emission timing according to a comparative example.
Fig. 8 is a block diagram illustrating a photoacoustic image generation apparatus according to a second embodiment of the invention.
Fig. 9 is a block diagram illustrating an operation procedure of the photoacoustic image generation apparatus according to the second embodiment.
Fig. 10 is a diagram illustrating a modified example of the driving means.
Fig. 11 is a graph illustrating molecular absorption coefficients of oxygenated hemoglobin and deoxygenated hemoglobin depending on light wavelengths.
Fig. 12 illustrates change characteristics of the transmission wavelength for the rotational displacement of the birefringent filter.

### DESCRIPTION OF THE PREFERRED EMBODIMENTS

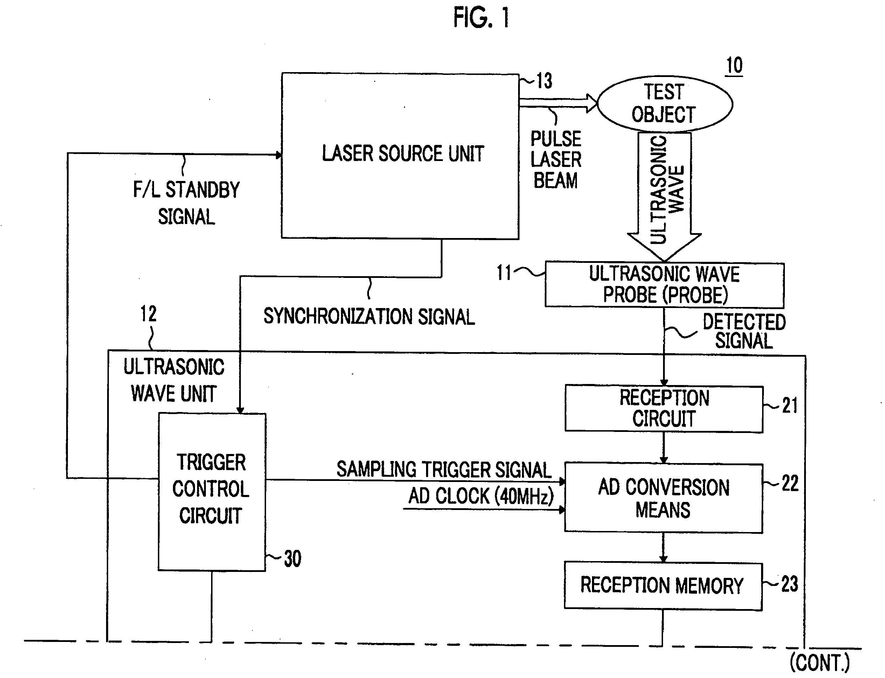

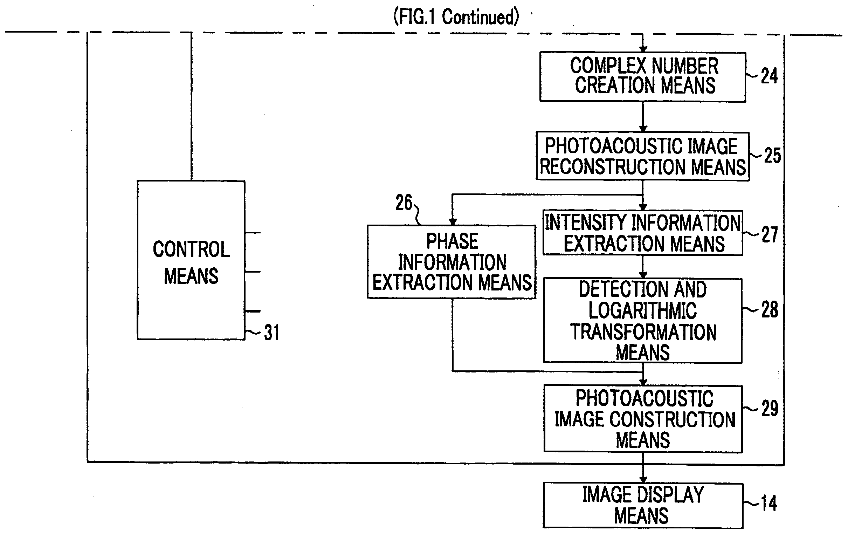

Hereinafter, embodiments of the invention will be described in detail with reference to the accompanying drawings. Meanwhile, in examples of the invention, ultrasonic waves are used as acoustic waves, but the acoustic waves may be acoustic waves having an audible frequency by selecting an appropriate frequency according to an object to be tested or measurement conditions. Fig. 1 illustrates a photoacoustic image generation apparatus according to a first embodiment of the invention. A photoacoustic image generation apparatus 10 includes an ultrasonic wave probe (probe) 11, an ultrasonic wave unit 12, and a laser source unit 13. The laser source unit 13 emits a pulse laser beam with which an object is to be irradiated. The laser source unit 13 emits a plurality of different pulse laser beams. Hereinafter, a description will be mainly given on the assumption that the laser source unit 13 switches and emits a pulse laser beam having a first wavelength and a pulse laser beam having a second wavelength.

For example, a wavelength of approximately 750 nm is considered as the first wavelength (center wavelength), and a wavelength of approximately 800 nm is considered as the second wavelength. First, referring to Fig. 11 described above, a molecular absorption coefficient of oxygenated hemoglobin (hemoglobin combined with oxygen: oxy-Hb) which is contained in a large amount in an artery of a human at a wavelength of 750 nm is lower than a molecular absorption coefficient of that at a wavelength of 800 nm. On the other hand, a molecular absorption coefficient of deoxygenated hemoglobin (hemoglobin not combined with oxygen: deoxy-Hb) which is contained in a large amount in a vein at a wavelength of 750 nm is higher than a molecular absorption coefficient of that at a wavelength of 800 nm. It is possible to discriminate between a photoacoustic signal from the artery and a photoacoustic signal from the vein by examining whether a photoacoustic signal obtained at the wavelength of 750 nm is relatively larger or smaller than a photoacoustic signal obtained at the wavelength of 800 nm, using such a property.

The pulse laser beam emitted from the laser source unit 13 is guided to a probe 11 using light guiding means such as an optical fiber, and is irradiated toward an object from the probe 11. An irradiation position of the pulse laser beam is not particularly limited, and the pulse laser beam may be irradiated from any place other than the probe 11. Ultrasonic waves (acoustic waves) are generated within the object by a light absorber absorbing energy of the irradiated pulse laser beam. The probe 11 includes an ultrasonic wave detector. The probe 11 includes, for example, a plurality of ultrasonic wave detector elements (ultrasonic wave vibrators) which are arranged one-dimensionally, and the acoustic waves (photoacoustic signal) from the inside of the object are detected by the ultrasonic wave vibrators that are arranged one-dimensionally.

The ultrasonic wave unit 12 includes a reception circuit 21, AD conversion means 22, a reception memory 23, complex number creation means 24, photoacoustic image reconstruction means 25, phase information extraction means 26, intensity information extraction means 27, detection and logarithmic transformation means 28, photoacoustic image construction means 29, a trigger control circuit 30, and control means 31. The reception circuit 21 receives a photoacoustic signal detected by the probe 11. The AD conversion means 22, which is sampling means, samples the photoacoustic signal received by the reception circuit 21 to generate photoacoustic data which is digital data. The AD conversion means 22 samples the photoacoustic signal with a predetermined sampling period in synchronization with, for example, an AD clock signal which is input from the outside.

The AD conversion unit 22 stores photoacoustic data in the reception memory 23. The AD conversion unit 22 stores, in the reception memory 23, photoacoustic data corresponding to the respective wavelengths of the pulse laser beam emitted from the laser source unit 13. In other words, the AD conversion means 22 stores, in the reception memory 23, first photoacoustic data converted from a photoacoustic signal detected by the probe 11 when an object is irradiated with a pulse laser beam having a first wavelength and second photoacoustic data converted from a photoacoustic signal detected by the probe 11 when the object is irradiated with a second pulse laser beam

The complex number creation means 24 reads out the first photoacoustic data and the second photoacoustic data from the reception memory 23, and generates complex number data in which any one of the first photoacoustic data and the second photoacoustic data is set to a real part and the other one is set to an imaginary part. Hereinafter, a description will be given on the assumption that the complex number creation means 24 generates complex number data in which the first photoacoustic data is set to a real part and the second photoacoustic data is set to as an imaginary part.

The photoacoustic image reconstruction means 25 inputs the complex number data from the complex number creation means 24. The photoacoustic image reconstruction means 25 performs image reconstruction from the input complex number data using a Fourier transform method (FTA method). A well-known method of the related art which is disclosed in, for example, a document "Photoacoustic Image Reconstruction-A Quantitative Analysis" Jonathan I. Sperl et al. SPIE-OSA, Vol. 6631 663103 can be applied to the image reconstruction using the Fourier transform method. The photoacoustic image reconstruction means 25 inputs data indicating the reconstructed image through Fourier transformation to the phase information extraction means 26 and the intensity information extraction means 27.

The phase information extraction means 26 generates phase information indicating a magnitude relation between relative signal intensities of pieces of photoacoustic data corresponding to the respective wavelengths. In this embodiment, the phase information extraction means 26 sets the reconstructed image reconstructed by the photoacoustic image reconstruction means 25 to input data. In addition, the phase information extraction means generates, when the real part and the imaginary part are compared with each other, phase information indicating how relatively large either of the two parts is. For example, when the complex number data is expressed by X+iY, the phase information extraction means 26 generates the relation of θ=tan⁻¹(Y/X) as phase information. Meanwhile, when the relation of X=0 is satisfied, the relation of θ=90° is established. When first photoacoustic data (X) constituting the real part is equal to second photoacoustic data (Y) constituting the imaginary part, the phase information satisfies the relation of θ=45°. As the first photoacoustic data becomes relatively larger, the phase information becomes closer to the relation of θ=0°. As the second photoacoustic data becomes larger, the phase information becomes closer to the relation of θ=90°.

The intensity information extraction means 27 generates intensity information indicating signal intensity on the basis of the pieces of photoacoustic data corresponding to the respective wavelengths. In this embodiment, the intensity information extraction means 27 sets the reconstructed image reconstructed by the photoacoustic image reconstruction means 25 to input data, and generates the intensity information from the input data which is complex number data. For example, when the complex number data is expressed by X+iY, the intensity information extraction means 27 extracts (X²+Y²)^{1/2} as intensity information. The detection and logarithmic transformation means 28 generates an envelope of data indicating the intensity information extracted by the intensity information extraction means 27, and then widens a dynamic range by performing logarithmic transformation on the envelope.

The photoacoustic image construction means 29 inputs the phase information from the phase information extraction means 26, and inputs the intensity information after the detection and logarithmic transformation process from the detection and logarithmic transformation means 28. The photoacoustic image construction means 29 generates a photoacoustic image which is a distribution image of a light absorber, on the basis of the input phase information and intensity information. For example, the photoacoustic image construction means 29 determines luminance (gradation value) of each pixel in the distribution image of the light absorber, on the basis of the input intensity information. In addition, for example, the photoacoustic image construction means 29 determines color of each pixel (display color) in the distribution image of the light absorber, on the basis of the phase information. The photoacoustic image construction means 29 determines color of each pixel on the basis of the input phase information, for example, using the range of phases 0° to 90° in a color map associated with a predetermined color.

Here, since the range of phases 0° to 45° is a range in which the first photoacoustic data is larger than the second photoacoustic data, a generation source of a photoacoustic signal is considered to be a vein through which blood flows, the blood mainly containing deoxygenated hemoglobin in which the amount of absorption of a wavelength of 756 nm is greater than that of a wavelength of 798 nm. On the other hand, since the range of phases 45° to 90° is a range in which the second photoacoustic data is smaller than the first photoacoustic data, the generation source of the photoacoustic signal is considered to be an artery through which blood flows, the blood mainly containing oxygenated hemoglobin in which the amount of absorption of a wavelength of 756 nm is less than that of a wavelength of 798 nm.

Consequently, as the color map, a color map is used in which color gradually changes so as to become blue at the phase of 0° and to become white as the phase approaches 45° and in which color gradually changes so as to become red at the phase of 90° and to become white as the phase approaches 45°. In this case, in the photoacoustic image, a portion corresponding to the artery can be expressed by red, and a portion corresponding to the vein can be expressed by blue. Color coding between the portion corresponding to the artery and the portion corresponding to the vein has only to be performed on the basis of the phase information, without using the intensity information. The image display means 14 displays the photoacoustic image generated by the photoacoustic image construction means 29 on a display screen.

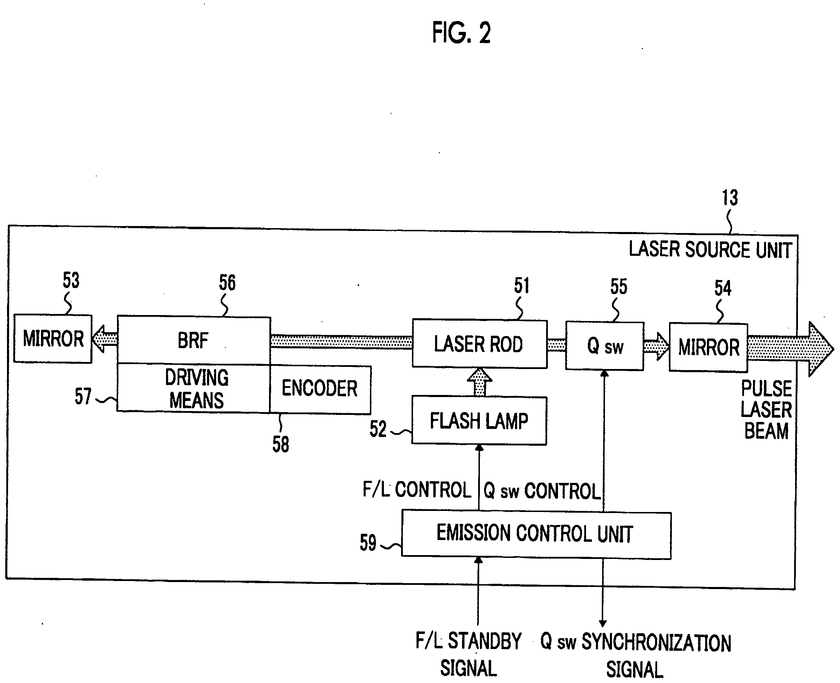

Subsequently, a configuration of the laser source unit 13 will be described in detail. Fig. 2 illustrates a configuration of the laser source unit 13. The laser source unit 13 includes a laser rod 51, a flash lamp 52, mirrors 53 and 54, a Q switch 55, a birefringent filter (BRF) 56, driving means 57, rotational displacement detection means 58, and an emission control unit 59. The laser rod 51 is a laser medium. For example, alexandrite crystal can be used as the laser rod 51. The flash lamp 52 is an excitation light source, and the laser rod 51 is irradiated with excitation light. Any of light sources other than the flash lamp 52 may be used as the excitation light source.

The mirrors 53 and 54 face each other with the laser rod 51 interposed therebetween, and an optical resonator is constituted by the mirrors 53 and 54. The mirror 54 is assumed to be the output side. The Q switch 55 and the birefringent filter 56 are inserted into the optical resonator. An insertion loss within the optical resonator rapidly changes from a high loss (low Q) to a low loss (high Q) by the Q switch 55, and thus a pulse laser beam can be obtained. The birefringent filter 56 changes a transmission wavelength in association with rotational displacement and changes an oscillation wavelength of the optical resonator. When the birefringent filter 56 is rotationally displaced in a fixed direction, the oscillation wavelength of the optical resonator changes to a sawtooth wave pattern for the rotational displacement of the birefringent filter 56.

The driving means 57 reciprocatively rotates the birefringent filter 56 in a predetermined range including a position at which the change characteristics of the transmission wavelength for the rotational displacement of the birefringent filter 56 are discontinuous. For example, when the birefringent filter 56 changes the transmission wavelength in a range of 740 nm to 810 nm, if the birefringent filter 56 is rotated in a certain direction, the transmission wavelength becomes longer at a fixed rate (gradient) with respect to the rotational displacement from 740 nm. When the transmission wavelength reaches 810 nm, the transmission wavelength returns to 740 nm, and then repeats such change characteristics of the transmission wavelength for the rotational displacement. A rotational displacement position returning from the wavelength of 810 nm to the wavelength of 740 nm is equivalent to a position at which the change characteristics of the transmission wavelength for the rotational displacement are discontinuous.

The rotational displacement detection means 58 detects the rotational displacement of the birefringent filter 56. The rotational displacement detection means 58 detects the rotational displacement of the birefringent filter 56 using, for example, a rotating plate with a slit which is mounted to a rotation axis of the birefringent filter 56 and a transmission photointerrupter.

The emission control unit 59 outputs a flash lamp trigger signal to the flash lamp 52, and irradiates the laser rod 51 with excitation light from the flash lamp 52. After the irradiation with the excitation light, the emission control unit 59 outputs a Q switch trigger signal to the Q switch 55 at a timing when the rotational displacement position of the birefringent filter 56 is set to a position corresponding to a wavelength of a pulse laser beam to be emitted. In addition, the emission control unit 59 outputs a Q switch synchronization signal to the ultrasonic wave unit 12 in accordance with an output timing of the Q switch trigger signal. The Q switch 55 rapidly changes the insertion loss within the optical resonator from a high loss to a low loss (Q switch is turned on) in response to the Q switch trigger signal, and thus the pulse laser beam is emitted from the mirror 54 on the output side.

Referring back to Fig. 1, the control means 31 controls each unit within the ultrasonic wave unit 12. The trigger control circuit 30 outputs a flash lamp standby signal for controlling the emission of the flash lamp 52 to the laser source unit 13. The trigger control circuit 30 receives the current rotational displacement position of the birefringent filter 56 which is detected by the rotational displacement detection means 58, from, for example, the laser source unit 13, and outputs the flash lamp standby signal at a timing based on the received rotational displacement position.

The trigger control circuit 30 inputs a Q switch synchronization signal indicating a timing at which the Q switch is turned on, that is, a laser emission timing, from the laser source unit 13. When the trigger control circuit 30 receives the Q switch synchronization signal, the trigger control circuit outputs a sampling trigger signal (AD trigger signal) to the AD conversion means 22. The AD conversion means 22 starts to sample a photoacoustic signal on the basis of the sampling trigger signal.

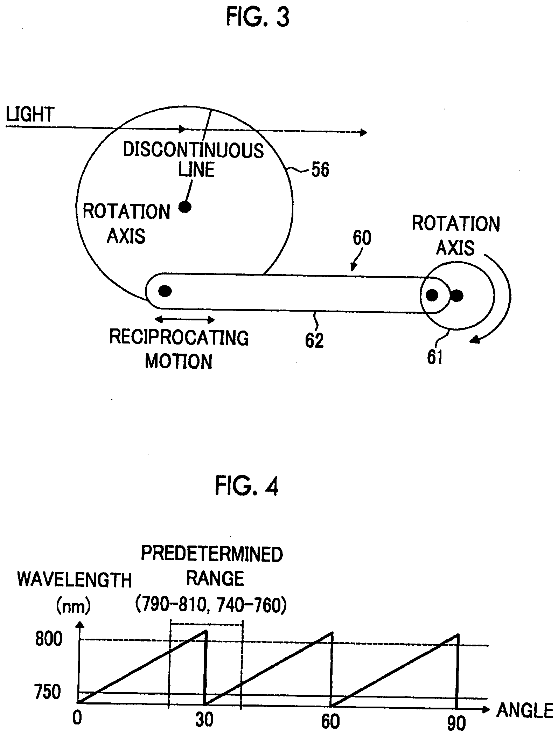

Fig. 3 illustrates a configuration example of the driving means 57 that reciprocatively rotates the birefringent filter 56. The driving means 57 includes a circularly-moving body (disc) 61 that circularly moves and a connecting rod 62 that connects the disc 61 and the birefringent filter 56 with each other. A crank mechanism 60 converting the circular motion of the disc 61 into a reciprocating rotational motion of the birefringent filter 56 is constituted by the disc 61 and the connecting rod 62. The disc 61 is rotated at a fixed speed around a rotation axis by, for example, a motor. A connecting point (supporting point) between the disc 61 and the connecting rod 62 is displaced in association with the rotation of the disc 61, and the birefringent filter 56 is reciprocatively rotated around the rotation axis in a predetermined range crossing the discontinuous point (discontinuous line) of the transmission wavelength change characteristics by the displacement. It is possible to reciprocatively rotate the birefringent filter 56 smoothly within a predetermined range using the crank mechanism. Here, the fixed speed means a speed which is determined in advance, and is synonymous with a predetermined speed.

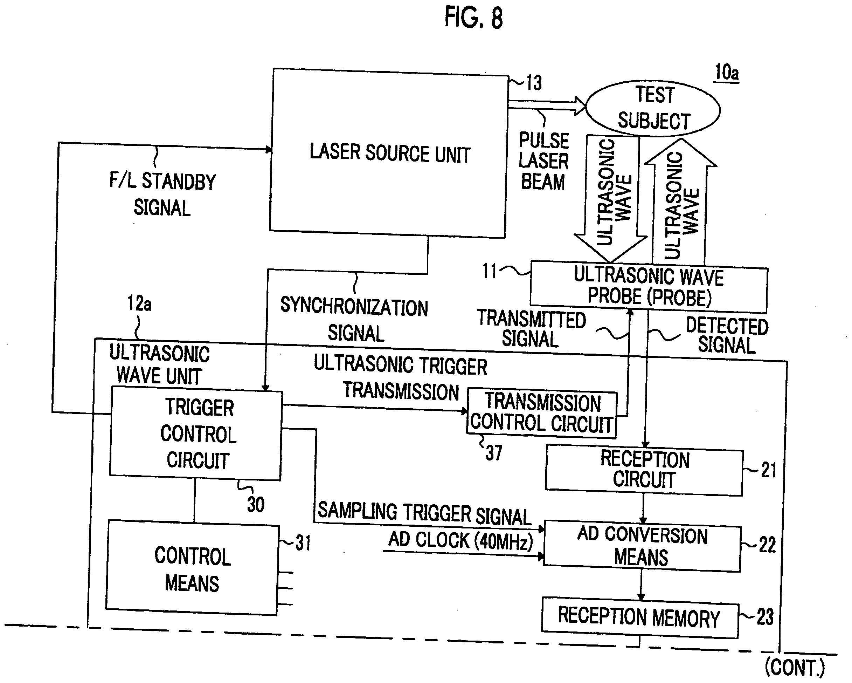

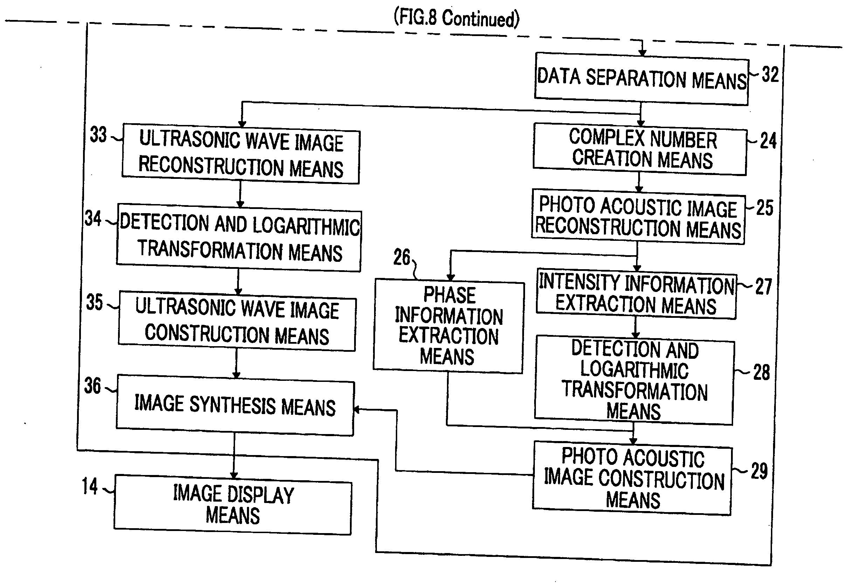

Fig. 4 illustrates change characteristics of a transmission wavelength (oscillation wavelength of optical resonator) for rotational displacement of the birefringent filter. For example, the same filter as the birefringent filter which has the change characteristics of the transmission wavelength illustrated in Fig. 8 can be used as the birefringent filter 56. The birefringent filter 56 repeats an FSR including a first wavelength (for example, 750 nm) and a second wavelength (for example, 800 nm) a plurality of times during one rotation in a fixed direction. Such a birefringent filter 56 is reciprocatively rotated in the predetermined range, shown by a dashed line in Fig. 4, including the discontinuous point of the transmission wavelength change characteristics.

For example, the birefringent filter 56 is reciprocatively rotated in a range in which the transmission wavelength changes from 790 nm to 810 nm with the increase in rotational displacement position, passes through the discontinuous point at which the transmission wavelength changes from 810 nm to 740 nm, and then changes from 740 nm to 760 nm. In this case, the predetermined range including the position at which the change characteristics of the oscillation wavelength are set to be a discontinuous point means a range in which the transmission wavelength changes from 790 nm to 810 nm with the increase in rotational displacement position, passes through the discontinuous point at which the transmission wavelength changes from 810 nm to 740 nm, and then changes from 740 nm to 760 nm. In the predetermined range in which the birefringent filter 56 is reciprocatively rotated, a position (first rotational displacement position) to which the birefringent filter is rotated by a certain amount in a first rotation direction (direction in which rotational displacement position increases), from the rotational displacement position (for example, 30 degrees) at which the transmission wavelength change characteristics are set to be a discontinuous point, is a rotational displacement position corresponding to the first wavelength (750 nm), and a position (second rotational displacement position) to which the birefringent filter is rotated by a certain amount in a second direction (direction in which rotational displacement position decreases) is a rotational displacement position corresponding to the second wavelength (800 nm). It is preferable that the predetermined range in which the birefringent filter 56 is reciprocatively rotated be smaller than a range of the rotational displacement of the birefringent filter which corresponds to one free spectral range.

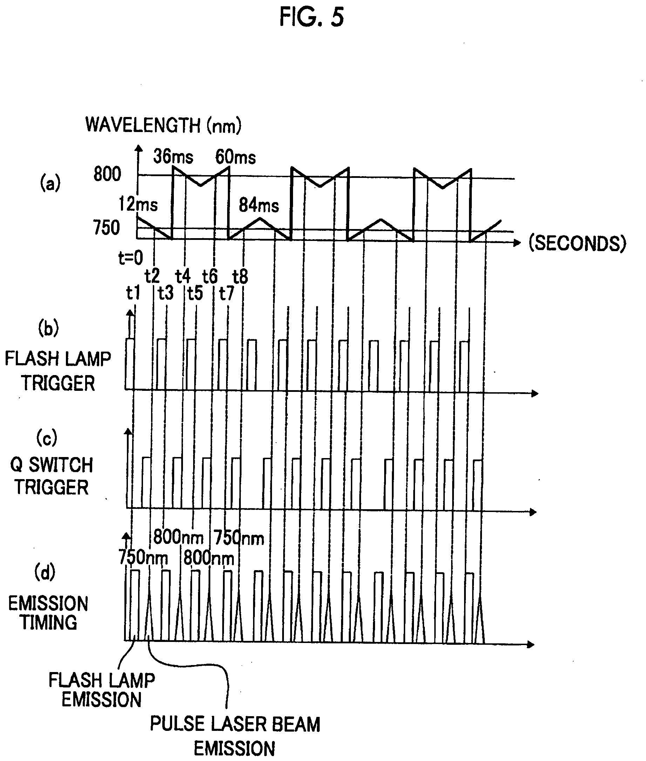

Fig. 5 is a timing chart illustrating various types of triggers and an emission timing. (a) of Fig. 5 illustrates oscillation wavelength characteristics (transmission wavelength characteristics of the birefringent filter 56) of an optical resonator with respect to change of time. (b) of Fig. 5 illustrates a flash lamp trigger, and (c) of Fig. 5 illustrates a Q switch trigger. (d) of Fig. 5 illustrates an emission timing of a flash lamp and an emission timing of a pulse laser beam. Meanwhile, in Fig. 5, for the purpose of simplifying the description, a description is given on the assumption that the flash lamp 52 and the Q switch 55 instantaneously respond to a trigger, but actually a delay time is present. However, since the delay is approximately several µ seconds to 100 µ seconds, the delay is negligible.

For example, the birefringent filter 56 is reciprocatively rotated at the same angular velocity as that when the birefringent filter is rotated once per second in a fixed direction. First, it is assumed that the birefringent filter 56 is rotationally displaced in a second rotation direction (negative direction) from the rotational displacement position corresponding to the wavelength 760 nm ((a) of Fig. 5). A time when the birefringent filter 56 starts to be rotationally displaced from the rotational displacement position corresponding to the wavelength 760 nm is set to a reference time t=0.

The trigger control circuit 30 of the ultrasonic wave unit 12 outputs the flash lamp standby signal in order to cause the pulse laser beam having a wavelength of 750 nm to be emitted from the laser source unit 13. When the emission control unit 59 of the laser source unit 13 receives the flash lamp standby signal, the emission control unit outputs the flash lamp trigger signal to the flash lamp 52 at time t1 ((b) of Fig. 5), and turns on the flash lamp 52 ((d) of Fig. 5). Thereafter, the emission control unit 59 outputs the Q switch trigger signal at time t2=12 ms when the rotational displacement position of the birefringent filter 56 is set to the position corresponding to the wavelength of 750 nm ((c) of Fig. 5), and turns on the Q switch 55, thereby causing the pulse laser beam having a wavelength of 750 nm to be emitted from the optical resonator.

The birefringent filter 56 is rotationally displaced across the discontinuous point of the transmission wavelength change characteristics ((a) of Fig. 5). The trigger control circuit 30 of the ultrasonic wave unit 12 outputs the flash lamp standby signal in order to cause a pulse laser beam having a wavelength of 800 nm to be emitted from the laser source unit 13. When the emission control unit 59 receives the flash lamp standby signal, the emission control unit outputs the flash lamp trigger signal to the flash lamp 52 at time t3 ((b) of Fig. 5), and turns on the flash lamp 52 ((d) of Fig. 5). Thereafter, the emission control unit 59 outputs the Q switch trigger signal at time t4=36 ms when the rotational displacement position of the birefringent filter 56 is set to the position corresponding to the wavelength of 800 nm ((c) of Fig. 5), and turns on the Q switch 55, thereby causing the pulse laser beam having a wavelength of 800 nm to be emitted from the optical resonator.

When the birefringent filter 56 is rotationally displaced up to a position corresponding to a wavelength of 790 nm, the driving means 57 reverses a rotation direction to rotationally displace the birefringent filter 56 in a first direction (positive direction) from the position corresponding to the wavelength of 790 nm ((a) of Fig. 5). The trigger control circuit 30 outputs the flash lamp standby signal in order to cause the pulse laser beam having a wavelength of 800 nm to be emitted from the laser source unit 13. When the emission control unit 59 receives the flash lamp standby signal, the emission control unit outputs the flash lamp trigger signal to the flash lamp 52 at time t5 ((b) of Fig. 5), and turns on the flash lamp 52 ((d) of Fig. 5). Thereafter, the emission control unit 59 outputs the Q switch trigger signal at time t6=60 ms when the rotational displacement position of the birefringent filter 56 is set to the position corresponding to the wavelength of 800 nm ((c) of Fig. 5), and turns on the Q switch 55, thereby causing the pulse laser beam having a wavelength of 800 nm to be emitted from the optical resonator.

The birefringent filter 56 is rotationally displaced across the discontinuous point of the transmission wavelength change characteristics in a direction opposite to the previous direction ((a) of Fig. 5). The trigger control circuit 30 of the ultrasonic wave unit 12 outputs the flash lamp standby signal in order to cause the pulse laser beam having a wavelength of 750 nm to be emitted from the laser source unit 13. When the emission control unit 59 receives the flash lamp standby signal, the emission control unit outputs the flash lamp trigger signal to the flash lamp 52 at time t7 ((b) of Fig. 5), and turns on the flash lamp 52 ((d) of Fig. 5). Thereafter, the emission control unit 59 outputs the Q switch trigger signal at time t8=84 ms when the rotational displacement position of the birefringent filter 56 is set to the position corresponding to the wavelength of 750 nm ((c) of Fig. 5), and turns on the Q switch 55, thereby causing the pulse laser beam having a wavelength of 750 nm to be emitted from the optical resonator.

Hereinafter, similarly, the emission control unit 59 turns on the flash lamp 52 to perform laser excitation while the driving means 57 reciprocatively rotates the birefringent filter 56, and then turns on the Q switch at the rotational displacement positions corresponding to the respective wavelengths, thereby causing the pulse laser beams having the first wavelength, the second wavelength, the second wavelength, and the first wavelength to be sequentially emitted. An interval of emission between the pulse laser beams having the respective wavelengths is set to approximately 48 ms equivalent to one cycle of the reciprocating rotational motion of the birefringent filter 56. In addition, a switching time of the wavelength is set to approximately 24 ms equivalent to a half cycle of the reciprocating rotational motion of the birefringent filter 56. The laser source unit 13 emits pulse laser beams having a set of wavelengths of 750 nm and 800 nm in units of twenty-one sets (a total of forty-two sets) per second (42 Hz operation).

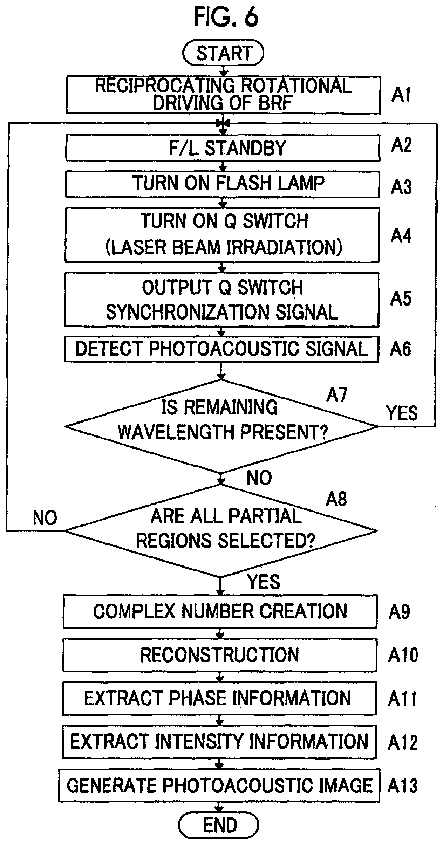

Fig. 6 illustrates an operation procedure of the photoacoustic image generation apparatus 10. Herein, a description is given on the assumption that a region of an object which is irradiated with a laser beam is divided into a plurality of partial regions. The driving means 57 reciprocatively rotates the birefringent filter 56, for example, in a range, including a discontinuous point (changing point from 740 nm to 810 nm), which corresponds to a transmission wavelength of 740 nm to 760 nm and a transmission wavelength of 790 nm to 810 nm, prior to the irradiation with the pulse laser beam with respect to the object (step A1). For example, the driving means 57 rotates the birefringent filter 56 in a negative direction from the position corresponding to the transmission wavelength 760 nm. At this time, for example, the driving means 57 rotates the birefringent filter at the same angular velocity as that in a case where the birefringent filter 56 is rotated once per second.

When the photoacoustic signal is ready to be received, the trigger control circuit 30 outputs a flash lamp standby signal to the laser source unit 13 at a predetermined timing in order to cause the pulse laser beam having a first wavelength (750 nm) to be emitted (step A2). When the flash lamp standby signal is input, the emission control unit 59 of the laser source unit 13 turns on the flash lamp 52 to cause the laser rod 51 to start to be excited (step A3). The emission control unit 59 turns on the flash lamp 52, for example, at a timing calculated back from a timing at which the rotational displacement position of the birefringent filter 56 is set to the position corresponding to the wavelength of 750 nm.

After the flash lamp 52 is turned on, the emission control unit 59 turns on the Q switch 55 at a timing when the rotational displacement position of the birefringent filter 56 is set to the position corresponding to the transmission wavelength of 750 nm (step A4). The laser source unit 13 emits the pulse laser beam having a wavelength of 750 nm by the Q switch 55 being turned on. The emission control unit 59 outputs a Q switch synchronization signal to the ultrasonic wave unit 12 in synchronization with the timing at which the Q switch 55 is turned on (step A5).

The pulse laser beam having a wavelength of 750 nm which is emitted from the laser source unit 13 is guided to, for example, the probe 11, and a first partial region of the object is irradiated with the pulse laser beam from the probe 11. A light absorber absorbs energy of the irradiated pulse laser beam within the object, and thus a photoacoustic signal is generated. The probe 11 detects the photoacoustic signal generated within the object. The photoacoustic signal detected by the probe 11 is received by the reception circuit 21.

After the flash lamp trigger signal is output, the trigger control circuit 30 stands by until the reception of the Q switch synchronization signal. When the trigger control circuit 30 receives the Q switch synchronization signal, the trigger control circuit outputs a sampling trigger signal to the AD conversion means 22. The AD conversion means 22 samples the photoacoustic signal received by the reception circuit 21 with a predetermined sampling period (step A6). The photoacoustic signal sampled by the AD conversion means 22 is stored as first photoacoustic data in the reception memory 23.

The control means 31 determines whether a remaining wavelength is present, in other words, whether the pulse laser beams having all the wavelengths to be emitted have been emitted (step A7). When a remaining wavelength is present, the process returns to step A2 in order to emit the pulse laser beam having the next wavelength, and the flash lamp standby signal is output to the laser source unit 13 from the trigger control circuit 30. In step A3, the emission control unit 59 turns on the flash lamp 52, and in step A4, the emission control unit turns on the Q switch 55 at a timing when the birefringent filter 56 is set to be at the rotational displacement position corresponding to the second wavelength (800 nm) to cause the pulse laser beam to be emitted.

The pulse laser beam having a wavelength of 800 nm which is emitted from the laser source unit 13 is guided to, for example, the probe 11, and the first partial region of the object is irradiated with the pulse laser beam from the probe 11. The probe 11 detects a photoacoustic signal generated by the light absorber within the object absorbing the pulse laser beam having a wavelength of 800 nm. When the trigger control circuit 30 receives the Q switch synchronization signal, the trigger control circuit outputs the sampling trigger signal to the AD conversion means 22, and the AD conversion means 22 samples the photoacoustic signal in step A6. The photoacoustic signal sampled by the AD conversion means 22 is stored as second photoacoustic data in the reception memory 23. The photoacoustic image generation apparatus 10 performs step A1 to step A6 on each wavelength, and irradiates the object with the pulse laser beams having the respective wavelengths, thereby detecting the photoacoustic signal from the object.

When the control means 31 determines in step A7 that a remaining wavelength is not present, the control means determines whether all the partial regions have been selected (step A8). When the partial region to be selected remains, the process returns to step A2. The photoacoustic image generation apparatus 10 performs step A2 to step A7 on each partial region, sequentially irradiates each partial region with pulse laser beams having the wavelengths (750 nm and 800 nm), and stores the first photoacoustic data and the second photoacoustic data which correspond to each partial region, in the reception memory 23. The order of wavelengths of the pulse laser beams with which the object are irradiated varies depending on a rotation direction of the birefringent filter 56. The laser source unit 13 repeatedly emits the pulse laser beams in the order, for example, 750 nm, 800 nm, 800 nm, and 750 nm. When the irradiation with the pulse laser beam and the detection of the photoacoustic signal are performed on all the partial regions, photoacoustic data required to generate a photoacoustic image of one frame is gathered.

When the control means 31 determines in step A8 that all the partial regions have been selected, the process proceeds to the generation of the photoacoustic image. The complex number creation means 24 reads out the first photoacoustic data and the second photoacoustic data from the reception memory 23, and generates complex number data in which first photoacoustic image data is set to a real part and second photoacoustic image data is set to an imaginary part (step A9). The photoacoustic image reconstruction means 25 performs image reconstruction from the complex number data generated in step A9, using a Fourier transform method (FTA method) (step A10).

The phase information extraction means 26 extracts phase information from the reconstructed complex number data (reconstructed image) (step A11). For example, when the reconstructed complex number data is expressed by X+iY, the phase information extraction means 26 extracts the relation of θ=tan⁻¹(Y/X) as the phase information (however, when the relation of X=0 is satisfied, the relation of θ=90° is satisfied). The intensity information extraction means 27 extracts intensity information from the reconstructed complex number data (step A12). For example, when the reconstructed complex number data is expressed by X+iY, the intensity information extraction means 27 extracts (X²+Y²)^{1/2} as the intensity information.

The detection and logarithmic transformation means 28 performs a detection and logarithmic transformation process on the intensity information extracted in step A12. The photoacoustic image construction means 29 generates a photoacoustic image on the basis of the phase information extracted in step A11 and the performing of the detection and logarithmic transformation process, on the intensity information extracted in step A12 (step A13). For example, the photoacoustic image construction means 29 generates the photoacoustic image by determining luminance (gradation value) of each pixel in a distribution image of a light absorber on the basis of the intensity information and by determining color of each pixel on the basis of the phase information. The generated photoacoustic image is displayed on the image display means 14.

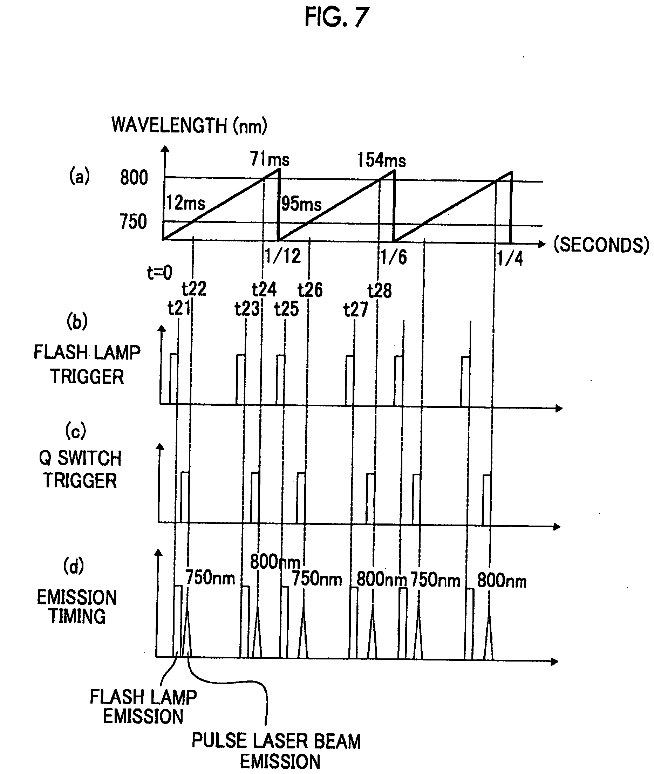

Here, as a comparative example, a case where the birefringent filter is rotated at a fixed speed in a predetermined direction is considered. Fig. 7 is a timing chart illustrating various types of triggers and an emission timing according to a comparative example. (a) of Fig. 7 illustrates oscillation wavelength characteristics (transmission wavelength characteristics of the birefringent filter 56) of an optical resonator with respect to change of time. (b) of Fig. 7 illustrates a flash lamp trigger, and (c) of Fig. 7 illustrates a Q switch trigger. (d) of Fig. 7 illustrates an emission timing of a flash lamp and an emission timing of a pulse laser beam. Meanwhile, in Fig. 7, for the purpose of simplifying the description, a description is given on the assumption that the flash lamp 52 and the Q switch 55 instantaneously respond to a trigger, but actually a delay time is present. However, since the delay is approximately several µ seconds to 100 µ seconds, the delay is negligible.

As illustrated in Fig. 4, when the birefringent filter 56 repeating an FSR twelve times for one rotation is rotated once per second, the birefringent filter 56 repeats the FSR twelve times per second ((a) of Fig. 7). First, it is assumed that the birefringent filter 56 is rotationally displaced in a first rotation direction (positive direction) from a rotational displacement position corresponding to a wavelength of 740 nm. A flash lamp trigger signal is output to the flash lamp 52 at time t21 ((b) of Fig. 7), and the flash lamp 52 is turned on ((d) of Fig. 7). Thereafter, a Q switch trigger signal is output at time t22=12 ms when the rotational displacement position of the birefringent filter 56 is set to a position corresponding to a wavelength of 750 nm ((c) of Fig. 7), and the Q switch 55 is turned on, and thus it is possible to cause a pulse laser beam having a wavelength of 750 nm to be emitted from the optical resonator.

Subsequently, the flash lamp trigger signal is output to the flash lamp 52 at time t23 ((b) of Fig. 7), and the flash lamp 52 is turned on ((d) of Fig. 7). Thereafter, the Q switch trigger signal is output at time t24=71 ms when the rotational displacement position of the birefringent filter 56 is set to a position corresponding to a wavelength of 800 nm ((c) of Fig. 7), and the Q switch 55 is turned on, and thus it is possible to cause a pulse laser beam having a wavelength of 800 nm to be emitted from the optical resonator. Hereinafter, similarly, the flash lamp trigger signal is output to the flash lamp 52 at time t25 and time t27, and the Q switch trigger signal is output to the Q switch 55 at time t26 and time t28, and thus it is possible to alternately emit the pulse laser beams having wavelengths of 750 nm and 800 nm.

In an example of Fig. 7, an interval of emission between the pulse laser beams having the respective wavelengths is equal to a repetition period of the FSR and is set to approximately 83 ms. In addition, a switching time of the wavelength is set to approximately 59 ms in the switching from the wavelength of 750 nm to the wavelength of 800 nm, and is set to approximately 24 ms in the switching from the wavelength of 800 nm to the wavelength of 750 nm.

Comparing Fig. 5 and Fig. 7, in this embodiment, the birefringent filter 56 is reciprocatively rotated in a predetermined range including the discontinuous point, and thus it is seen that the speed of the switching from the wavelength of 750 nm to the wavelength of 800 nm can be increased. Specifically, in Fig. 7, two pulse laser beams having the wavelengths of 750 nm and 800 nm are emitted for 83 ms. On the other hand, in Fig. 5, it is possible to emit two pulse laser beams having the wavelengths of 750 nm and 800 nm for 48 ms and to realize speeding up by approximately 1.7 times. In addition, in Fig. 7, the switching from the wavelength of 750 nm to the wavelength of 800 nm and the switching from the wavelength of 800 nm to the wavelength of 750 nm are performed at unequal intervals. On the other hand, in Fig. 5, the predetermined range in which the birefringent filter is reciprocatively rotated is appropriately set, and thus the switching time of the wavelengths of the pulse laser beams can be set to a fixed time. A trigger interval for the flash lamp 52 and the Q switch 55 is set to be fixed, and thus it is possible to prevent waste of time and to prevent a load from being applied to a lamp.

In this embodiment, the birefringent filter 56 is reciprocatively rotated in a predetermined range including a discontinuous point of change characteristics of a transmission wavelength for the rotational displacement, and the Q switch 55 is turned on at a timing when the birefringent filter is set to be at a position corresponding to a wavelength of a pulse laser beam to be emitted, thereby causing the pulse laser beam to be emitted. A change in transmission wavelength with respect to the rotational displacement is great at the discontinuous point of the change characteristics of the transmission wavelength for the rotational displacement. By using this feature, it is possible to greatly change the transmission wavelength of the birefringent filter 56 as compared with a case where the birefringent filter is rotationally driven in a fixed direction, without increasing the amount of driving of the birefringent filter 56 per unit time. Therefore, it is possible to switch and emit a plurality of wavelengths at a high speed without increasing a rotation speed (angular velocity) of the birefringent filter.

In particular, it is preferable that the predetermined range in which the birefringent filter 56 is reciprocatively rotated be set to be smaller than a range of the rotational displacement of the birefringent filter 56 which corresponds to one FSR. In this case, when the angular velocity is set to be constant and, for example, wavelengths of 750 nm and 800 nm are used as one set, an interval of time between the respective sets can be made to be shorter than a time required for the rotational displacement of the birefringent filter 56 by the amount corresponding to one FSR. In addition, when the predetermined range in which the birefringent filter 56 is reciprocatively rotated is appropriately set depending on the position of the discontinuous point in the birefringent filter 56 and the rotational displacement positions corresponding to the respective wavelengths, the pulse laser beam can be emitted at fixed intervals.

In this embodiment, complex number data is generated in which one of the first photoacoustic data and the second photoacoustic data which are obtained at two wavelengths is set to a real part and the other is set to an imaginary part, and a reconstructed image is generated from the complex number data using a Fourier transform method. In this case, it is possible to effectively perform the reconstruction as compared with a case where the first photoacoustic data and the second photoacoustic data are separately reconstructed. Pulse laser beams having a plurality of different wavelengths is irradiated, and a photoacoustic signal (photoacoustic data) at the time of the irradiation with a pulse laser beam having each wavelength is used, and thus it is possible to perform functional imaging using optical absorption properties of the respective light absorbers being different from each other depending on wavelengths.

In addition, in this embodiment, for example, when a light irradiation region is divided into three partial regions, a first partial region is sequentially irradiated with a pulse laser beam having a first wavelength and a pulse laser beam having a second wavelength, and a second partial region is sequentially irradiated with the pulse laser beam having the first wavelength and the pulse laser beam having the second wavelength, and then a third partial region is sequentially irradiated with the pulse laser beam having the first wavelength and the pulse laser beam having the second wavelength. In this embodiment, a certain partial region is continuously irradiated with the pulse laser beam having the first wavelength and the pulse laser beam having the second wavelength, and then the irradiation proceeds to the next partial region. In this case, it is possible to shorten time between the irradiation with the pulse laser beam having the first wavelength and the irradiation with the pulse laser beam having the second wavelength at the same position, as compared with a case where three partial regions are irradiated with the laser beam having the first wavelength and are then irradiated with the pulse laser beam having the second wavelength. It is possible to suppress mismatching between the first photoacoustic data and the second photoacoustic data by shortening the time between the irradiation with the pulse laser beam having the first wavelength and the irradiation with the pulse laser beam having the second wavelength.

Subsequently, a second embodiment of the invention will be described. Fig. 8 illustrates a photoacoustic image generation apparatus according to the second embodiment of the invention. In a photoacoustic image generation apparatus 10a of this embodiment, an ultrasonic wave unit 12a includes data separation means 32, ultrasonic image reconstruction means 33, detection and logarithmic transformation means 34, ultrasonic image construction means 35, image synthesis means 36, and a transmission control circuit 37, in addition to the configuration of the ultrasonic wave unit 12 in the photoacoustic image generation apparatus 10 according to the first embodiment which is illustrated in Fig. 1. The photoacoustic image generation apparatus 10 of this embodiment is different from that in the first embodiment in that the apparatus generates an ultrasonic image in addition to a photoacoustic image. Other parts may be the same as those in the first embodiment.

In this embodiment, a probe 11 outputs (transmits) ultrasonic waves to an object and detects (receives) reflected ultrasonic waves from the object with respect to the transmitted ultrasonic waves, in addition to the detection of a photoacoustic signal. A trigger control circuit 30 transmits an ultrasonic wave transmission trigger signal for instructing the transmission of ultrasonic waves to the transmission control circuit 37 at the time of the generation of an ultrasonic image. When the transmission control circuit 37 receives the trigger signal, the transmission control circuit causes ultrasonic waves to be transmitted from the probe 11. The probe 11 detects reflected ultrasonic waves from the object after the transmission of the ultrasonic waves.

The reflected ultrasonic waves detected by the probe 11 are input to AD conversion means 22 through a reception circuit 21. The trigger control circuit 30 transmits a sampling trigger signal to the AD conversion means 22 in accordance with the transmission timing of the ultrasonic waves, and starts to sample the reflected ultrasonic waves. The AD conversion means 22 stores sampling data of the reflected ultrasonic waves (reflected ultrasonic data) in the reception memory 23.

The data separation means 32 separates the reflected ultrasonic data stored in the reception memory 23 and first and second photoacoustic data from each other. The data separation means 32 transmits the reflected ultrasonic data to the ultrasonic image reconstruction means 33, and transmits the first and second photoacoustic data to complex number creation means 24. The generation of the photoacoustic image on the basis of the first and second photoacoustic data is the same as that in the first embodiment. The data separation means 32 inputs sampling data of the separated reflected ultrasonic waves to the ultrasonic image reconstruction means 33.

The ultrasonic image reconstruction means 33 generates pieces of data of lines of the ultrasonic image on the basis of reflected ultrasonic waves (sampling data thereof) which are detected by a plurality of ultrasonic vibrators of the probe 11. For example, the ultrasonic image reconstruction means 33 adds data from 64 ultrasonic vibrators of the probe 11 on the basis of a delay time depending on the position of the ultrasonic vibrator to generate data for one line (delay addition method). The ultrasonic image reconstruction means 33 may perform reconstruction using a circular back projection (CBP) method instead of the delay addition method. Alternatively, the ultrasonic image reconstruction means 33 may perform reconstruction using a Hough transform method or a Fourier transform method.

The detection and logarithmic transformation means 34 obtains an envelope of the pieces of data of the lines which are output by the ultrasonic image reconstruction means 33, and performs logarithmic transformation on the obtained envelope. The ultrasonic image construction means 35 generates an ultrasonic image on the basis of the data of the lines on which the logarithmic transformation is performed. The ultrasonic image reconstruction means 33, the detection and logarithmic transformation means 34, and the ultrasonic image construction means 35 constitute ultrasonic image generation means that generates an ultrasonic image on the basis of reflected ultrasonic waves.

The image synthesis means 36 synthesizes the photoacoustic image and the ultrasonic image. For example, the image synthesis means 36 performs image synthesis by superimposing the photoacoustic image and the ultrasonic image on each other. At this time, it is preferable that the image synthesis means 36 perform positioning so that corresponding points of the photoacoustic image and the ultrasonic image are set to be at the same position. The synthesized image is displayed on image display means 14. It is also possible to display the photoacoustic image and the ultrasonic image on the image display means 14 side by side without performing image synthesis, or to switch and display the photoacoustic image and the ultrasonic image.

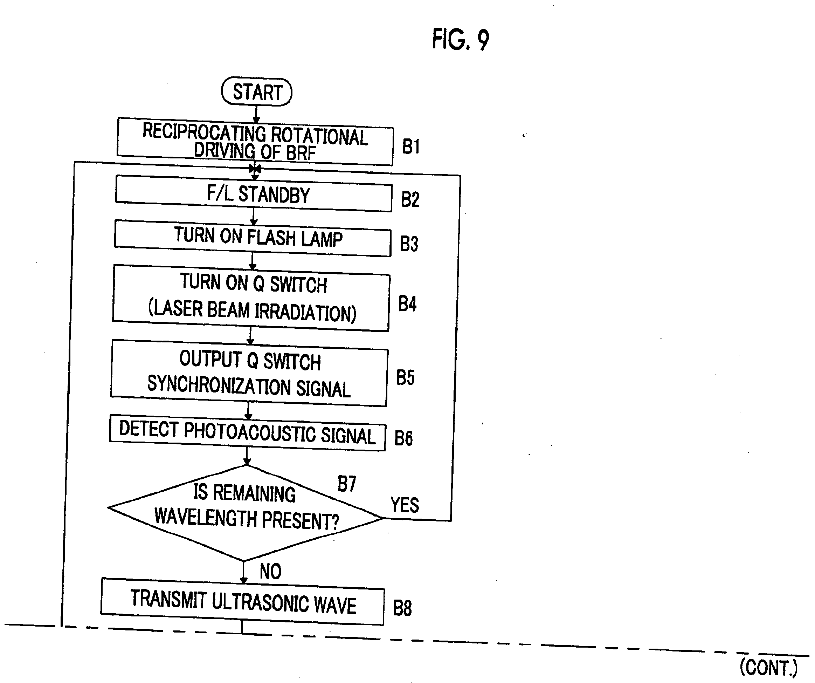

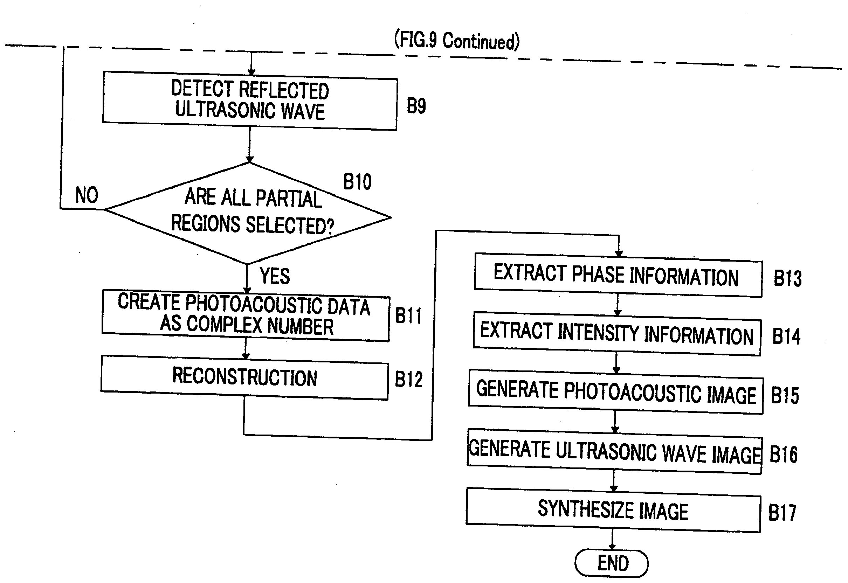

Fig. 9 illustrates an operation procedure of the photoacoustic image generation apparatus 10a. Hereinafter, a description is given on the assumption that a region of an object which is irradiated with a pulse laser beam is divided into a plurality of partial regions. The driving means 57 reciprocatively rotates the birefringent filter 56 in a predetermined range (step B1).

When a photoacoustic signal is ready to be received, the trigger control circuit 30 outputs a flash lamp trigger signal in order to cause a pulse laser beam having a first wavelength of 750 nm to be emitted (step B2). When the flash lamp standby signal is input, an emission control unit 59 of a laser source unit 13 turns on a flash lamp 52 to cause a laser rod 51 to start to be excited (step B3).

After the flash lamp 52 is turned on, the emission control unit 59 turns on a Q switch 55 at a timing when the rotational displacement position of the birefringent filter 56 is set to a position corresponding to a transmission wavelength of 750 nm (step B4). The laser source unit 13 emits a pulse laser beam having a wavelength of 750 nm by the Q switch 55 being turned on. The emission control unit 59 outputs a Q switch synchronization signal to the ultrasonic wave unit 12 in synchronization with the timing at which the Q switch 55 is turned on (step B5).

The pulse laser beam having a wavelength of 750 nm which is emitted from the laser source unit 13 is guided to, for example, the probe 11, and a first region of the object is irradiated with the pulse laser beam from the probe 11. A light absorber absorbs energy of the irradiated pulse laser beam within the object, and thus a photoacoustic signal is generated. The probe 11 detects the photoacoustic signal generated within the object. When the trigger control circuit 30 receives the Q switch synchronization signal, the trigger control circuit outputs a sampling trigger signal to the AD conversion means 22. The AD conversion means 22 receives the photoacoustic signal detected by the probe 11 through the reception circuit 21, and samples the photoacoustic signal with a predetermined sampling period (step B6). The photoacoustic signal sampled by the AD conversion means 22 is stored as first photoacoustic data in the reception memory 23.

The control means 31 determines whether a remaining wavelength is present, in other words, whether the pulse laser beams having all the wavelengths to be emitted have been emitted (step B7). When a remaining wavelength is present, the process returns to step B2 in order to emit the pulse laser beam having the next wavelength, and the flash lamp standby signal is output to the laser source unit 13 from the trigger control circuit 30. In step B3, the emission control unit 59 turns on the flash lamp 52, and in step B4, the emission control unit turns on the Q switch 55 at a timing when the birefringent filter 56 is set to be at the rotational displacement position corresponding to a second wavelength of 800 nm to cause the pulse laser beam to be emitted.

The pulse laser beam having a wavelength of 800 nm which is emitted from the laser source unit 13 is guided to, for example, the probe 11, and the first partial region of the object is irradiated with the pulse laser beam from the probe 11. The probe 11 detects the photoacoustic signal generated by the light absorber within the object absorbing the pulse laser beam having a wavelength of 800 nm. When the trigger control circuit 30 receives the Q switch synchronization signal, the trigger control circuit outputs the sampling trigger signal to the AD conversion means 22, and the AD conversion means 22 samples the photoacoustic signal in step B6. The photoacoustic signal sampled by the AD conversion means 22 is stored as second photoacoustic data in the reception memory 23. The photoacoustic image generation apparatus 10 performs step B1 to step B6 on each wavelength, and irradiates the object with the pulse laser beams having the respective wavelengths, thereby detecting the photoacoustic signal from the object. Here, step B1 to step B6 may be the same as step A1 to step A6 of Fig. 6.

When the control means 31 determines in step B7 that a remaining wavelength is not present, the process proceeds to the transmission and reception of ultrasonic waves. The trigger control circuit 30 transmits the ultrasonic waves to the object from the probe 11 through the transmission control circuit 37 (step B8). In step B8, the ultrasonic waves are transmitted to the same region as the partial region of the object which is irradiated with the pulse laser beam. The probe 11 detects reflected ultrasonic waves with respect to the transmitted ultrasonic waves (step B9). The detected reflected ultrasonic waves are sampled in the AD conversion means 22 through the reception circuit 21, and are stored as reflected ultrasonic data in the reception memory 23.

The control means 31 determines whether all the partial regions have been selected (step B10). When the partial region to be selected remains, the process returns to step B2. The photoacoustic image generation apparatus 10 performs step B2 to step B7 on each partial region, sequentially irradiates each partial region with pulse laser beams having the wavelengths (750 nm and 800 nm), and stores the first photoacoustic data and the second photoacoustic data in the reception memory 23. In addition, step B8 and step B9 are performed to store the reflected ultrasonic data in the reception memory 23. When the irradiation with the pulse laser beam, the detection of the photoacoustic signal, and the transmission and reception of the ultrasonic waves are performed on all the partial regions, data required to generate a photoacoustic image and an ultrasonic image of one frame is gathered.

When the control means 31 determines in step B10 that all the partial regions have been selected, the process proceeds to the generation of the photoacoustic image and the ultrasonic image. The data separation means 32 separates the first and second photoacoustic data and the reflected ultrasonic data from each other. The data separation means 32 transmits the separated first and second photoacoustic data to the complex number creation means 24, and transmits the reflected ultrasonic data to the ultrasonic image reconstruction means 33. The complex number creation means 24 generates complex number data in which first photoacoustic image data is set to a real part and second photoacoustic image data is set to an imaginary part (step B11). The photoacoustic image reconstruction means 25 performs image reconstruction from the complex number data generated in step B11, using a Fourier transform method (FTA method) (step B12).

The phase information extraction means 26 extracts phase information from the reconstructed complex number data (step B13). The intensity information extraction means 27 extracts intensity information from the reconstructed complex number data (step B14). The detection and logarithmic transformation means 28 performs a detection and logarithmic transformation process on the intensity information extracted in step B14. The photoacoustic image construction means 29 generates a photoacoustic image on the basis of the phase information extracted in step B13 and the performing of the detection and logarithmic transformation process on the intensity information extracted in step B14 (step B15). Here, step B11 to step B15 may be the same as step A9 to step A13 of Fig. 6.

The ultrasonic image reconstruction means 33 generates pieces of data of lines of the ultrasonic image using, for example, a delay addition method. The detection and logarithmic transformation means 34 obtains an envelope of the pieces of data of the lines which are output by the ultrasonic image reconstruction means 33, and performs logarithmic transformation on the obtained envelope. The ultrasonic image construction means 35 generates an ultrasonic image on the basis of the pieces of data of the lines on which the logarithmic transformation is performed (step B16). The image synthesis means 36 synthesizes the photoacoustic image and the ultrasonic image and displays the synthesized image on the image display means 14 (step B17).

In this embodiment, the photoacoustic image generation apparatus generates an ultrasonic image in addition to a photoacoustic image. It is possible to observe a portion not capable of being formed as an image in the photoacoustic image by referring to the ultrasonic image. Other effects are the same as those in the first embodiment.

Meanwhile, in the above-described embodiments, an example in which first photoacoustic data and second photoacoustic data are created as complex numbers has been described, but the first photoacoustic data and the second photoacoustic data may be separately reconstructed without being created as complex numbers. Phase information can be generated by comparing the first photoacoustic data (first reconstructed image) and the second photoacoustic data (second reconstructed image) which are separately reconstructed. In addition, the intensity information can be generated on the basis of signal intensity in the first reconstructed image and signal intensity in the second reconstructed image.

In the generation of a photoacoustic image, the number of wavelengths of a pulse laser beam with which an object is to be irradiated is not limited two, and the object may be irradiated with three or more pulse laser beams, and thus the photoacoustic image may be generated on the basis of pieces of photoacoustic data corresponding to the respective wavelengths. In this case, for example, the phase information extraction means 26 may generate a magnitude relation between relative signal intensities of the pieces of photoacoustic data corresponding to the respective wavelengths, as phase information. In addition, the intensity information extraction means 27 may generate the signal intensities in the pieces of photoacoustic data corresponding to the respective wavelengths, which are grouped into one, as intensity information.

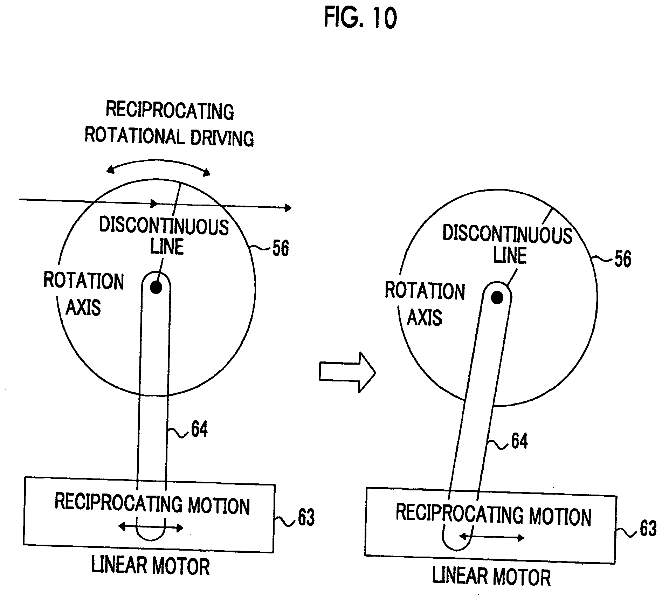

In Fig. 3, a description has been made on the assumption that the driving means 57 has a crank mechanism, but is not limited thereto. The driving means 57 may be means that reciprocatively rotates the birefringent filter 56 in a predetermined range, and the specific configuration thereof is not particularly limited. The birefringent filter 56 may be reciprocatively rotated using anything other than the crank mechanism. Fig. 10 illustrates a modified example of the driving means. The driving means according to the modified example includes a mechanism converting a linear reciprocating motion to a reciprocating rotational motion in a predetermined range. One end of a connecting rod 64 is connected to a linear motor 63, and the other end thereof is connected to the rotation axis of the birefringent filter 56. One end of the connecting rod 64 may be linearly reciprocated by the linear motor 63, and the birefringent filter 56 may be reciprocatively rotated in a predetermined range in association with the linear reciprocating motion. In addition, the birefringent filter 56 may be reciprocatively rotated using a cam mechanism.

As described above, the invention has been described on the basis of the preferred embodiments, a laser source unit, a control method thereof, a photoacoustic image generation apparatus, and a photoacoustic image generation method of the invention are not limited to those in the above-described embodiments, and various corrections and modifications made to the configurations of the above-described embodiments may also be included in the scope of the invention.

## Claims

1. A laser source unit (13) capable of emitting pulse laser beams of plurality of different wavelengths, the laser source unit comprising:
a laser rod (51);
an excitation light source (52) that irradiates the laser rod with excitation light;
an optical resonator that includes a pair of mirrors (53, 54) facing each other with the laser rod interposed therebetween;
a Q switch (55) which is inserted into the optical resonator;
a birefringent filter (56) which is inserted into the optical resonator and changes an oscillation wavelength of the optical resonator in association with rotational displacement of the birefringent filter;
driving means (57) that is configured to reciprocatively rotate the birefringent filter in a predetermined range including a position at which change characteristics of the oscillation wavelength for the rotational displacement of the birefringent filter are set to be a discontinuous point; and
an emission control unit (59) that is configured to irradiate the laser rod with excitation light from the excitation light source and is configured to turn on the Q switch, at a timing when a rotational displacement position of the birefringent filter is set to a position corresponding to the wavelength of the pulse laser beam to be emitted, to cause the pulse laser beam to be emitted.

2. The laser source unit (13) according to claim 1, wherein the driving means (57) includes a rotating body (61) that is configurated to circularly move and a rod (62) that is connecting the rotating body and the birefringent filter, and has a crank mechanism (60) configurated to convert a circular motion of the rotating body to a reciprocating rotational motion of the birefringent filter.

3. The laser source unit (13) according to claim 2, wherein the rotating body is configured to be rotated at a fixed speed.

4. The laser source unit (13) according to claim 1, wherein the driving means has a mechanism converting a linear reciprocating motion to a reciprocating rotational motion in a predetermined range.

5. The laser source unit (13) according to any one of claims 1 to 4,
wherein the plurality of wavelengths include first and second wavelengths, and
wherein the oscillation wavelength of the optical resonator is set to the first wavelength at a first rotational displacement position to which the birefringent filter is rotated in a first rotation direction by a first amount from the discontinuous point, and the oscillation wavelength of the optical resonator is set to the second wavelength at a second rotational displacement position to which the birefringent filter is rotated in a second direction, opposite to the first direction, by a second amount from the discontinuous point.

6. The laser source unit (13) according to any one of claims 1 to 5, wherein the birefringent filter is configured to repeat a free spectral range including the first wavelength and the second wavelength a plurality of times during one rotation in a fixed direction.

7. The laser source unit (13) according to claim 6, wherein the predetermined range in which the birefringent filter is reciprocatively rotated is smaller than a range of the rotational displacement of the birefringent filter which corresponds to one free spectral range.

8. The laser source unit (13) according to any one of claims 1 to 7, wherein the oscillation wavelength of the optical resonator changes to a sawtooth wave pattern for the rotational displacement of the birefringent filter.

9. A photoacoustic image generation apparatus comprising:
a laser source unit (13) according to any of claims 1 to 8;
sampling means that is configured to sample a photoacoustic signal, generated within an object when the object is irradiated with the pulse laser beam having a plurality of wavelengths, to generate pieces of photoacoustic data corresponding to the respective wavelengths;
phase information extraction means that is configured to generate phase information indicating a magnitude relation between relative signal intensities of the pieces of photoacoustic data corresponding to the respective wavelengths; and
photoacoustic image construction means that is configured to generate a photoacoustic image on the basis of the phase information.

10. The photoacoustic image generation apparatus (10) according to claim 9, further comprising intensity information extraction means that is configured to generate intensity information indicating signal intensity on the basis of the pieces of photoacoustic data corresponding to the respective wavelengths,
wherein the photoacoustic image construction means is configured to generate a gradation value of each pixel of the photoacoustic image on the basis of the intensity information and determines a display color of each pixel on the basis of the phase information.

11. The photoacoustic image generation apparatus (10) according to claim 10.
wherein the plurality of wavelengths include first and second wavelengths,
wherein the photoacoustic image generation apparatus further comprises:
complex number creation means that is configured to generate complex number data in which any one of first photoacoustic data corresponding to a photoacoustic signal, detected when irradiation with a pulse laser beam having the first wavelength is performed, and second photoacoustic data corresponding to a photoacoustic signal, detected when irradiation with a pulse laser beam having the second wavelength is performed, is set to a real part and the other one is set to an imaginary part; and
photoacoustic image reconstruction means that is configured to generate a reconstructed image from the complex number data using a Fourier transform method, and
wherein the phase information extraction means extracts the phase information from the reconstructed image, and the intensity information extraction means is configured to extract the intensity information from the reconstructed image.

12. The photoacoustic image generation apparatus (10) according to any one of claims 9 to 11.
wherein the sampling means is further configured to sample reflected acoustic waves with respect to acoustic waves transmitted to the object to generate reflected acoustic wave data, and
wherein the photoacoustic image generation apparatus further comprises acoustic wave image generation means that is configured to generate an acoustic wave image on the basis of the reflected acoustic wave data.

13. A control method of a laser source unit (13) capable of emitting pulse laser beams having a plurality of different wavelengths, the control method comprising:
a step of reciprocatively rotating, in a predetermined range, a birefringent filter (56) which is inserted into an optical resonator including a pair of mirrors (53, 54) facing each other with a laser rod (51) interposed therebetween and changes an oscillation wavelength of the optical resonator in association with rotational displacement of the birefringent filter; and
a step of irradiating the laser rod with excitation light, and after the irradiation with the excitation light, turning on a Q switch (55) inserted into the optical resonator, at a timing when a rotational displacement position of the birefringent filter is set to a position corresponding to the wavelength of the pulse laser beam to be emitted, to cause the pulse laser beam to be emitted.

14. A photoacoustic image generation method comprising:
further comprising
a step of sampling a photoacoustic signal generated within an object when the object is irradiated with the pulse laser beam having a plurality of wavelengths to generate pieces of photoacoustic data corresponding to the respective wavelengths;
a step of generating phase information indicating a magnitude relation between relative signal intensities of the pieces of photoacoustic data corresponding to the respective - wavelengths; and
a step of generating a photoacoustic image on the basis of the phase information.

## Patentansprüche

1. Laserquellen-Einheit (13), die in der Lage ist, gepulste Laserstrahlen mit einer Vielzahl von unterschiedlichen Wellenlängen zu emittieren, wobei die Laserquellen-Einheit umfasst:
einen Laserstab (51);
eine Anregungslichtquelle (52), die den Laserstab mit Anregungslicht bestrahlt;
einen optischen Resonator, der ein Paar von einander gegenüberstehenden Spiegeln (53, 54) umfasst, wobei der Laserstab dazwischen angeordnet ist;
einen Q-Switch (55), der in den optischen Resonator eingesetzt ist;
einen doppelbrechenden Filter (56), der in den optischen Resonator eingesetzt ist und eine Oszillationswellenlänge des optischen Resonators in Verbindung mit einer Rotations-Verschiebung des doppelbrechenden Filters verändert;
ein Ansteuermittel (57), das eingerichtet ist, den doppelbrechenden Filter in einem vorbestimmten Bereich hin und her zu rotieren, umfassend eine Position, an welcher eine Änderungscharakteristiken der Oszillationswellenlänge für die Rotations-Verschiebung des doppelbrechenden Filters als ein diskontinuierlicher Punkt eingestellt sind; und
eine Emissions-Steuereinheit (59), die eingerichtet ist, den Laserstab mit Anregungslicht von der Anregungslichtquelle zu bestrahlen, und eingerichtet ist, den Q-Switch zu einem Zeitpunkt anzuschalten, wenn eine Rotations-Verschiebungsposition des doppelbrechenden Filters auf eine Position eingestellt ist, die der zu emittierenden Wellenlänge des gepulsten Laserstrahls entspricht, um zu veranlassen, dass der gepulste Laserstrahl emittiert wird.

2. Laserquellen-Einheit (13) nach Anspruch 1, wobei das Ansteuermittel (57) einen Rotationskörper (61), der eingerichtet ist, sich zirkular zu bewegen und einen Stab (62) umfasst, der den Rotationskörper und den doppelbrechenden Filter verbindet, und einen Kurbelmechanismus (60) aufweist, der eingerichtet ist eine Zirkularbewegung des Rotationskörpers in eine Pendel-Rotationsbewegung des doppelbrechenden Filters zu konvertieren.

3. Laserquellen-Einheit (13) nach Anspruch 2, wobei der Rotationskörper eingerichtet ist, bei fester Geschwindigkeit rotiert zu werden.

4. Laserquellen-Einheit (13) nach Anspruch 1, wobei das Ansteuermittel einen Mechanismus aufweist, der in einem vorbestimmten Bereich eine lineare Pendelbewegung in eine Pendel-Rotationsbewegung konvertiert.

5. Laserquellen-Einheit (13) nach einem der Ansprüche 1 bis 4,
wobei die Vielzahl von Wellenlängen erste und zweite Wellenlängen umfasst, und
wobei die Oszillationswellenlänge des optischen Resonators an einer ersten Rotations-Verschiebungsposition, zu welcher der doppelbrechende Filter in einer ersten Rotationsrichtung von dem diskontinuierlichen Punkt um einen ersten Betrag rotiert ist, auf die erste Wellenlänge eingestellt ist, und die Oszillationswellenlänge des optischen Resonators an einer zweiten Rotations-Verschiebungsposition, zu welcher der doppelbrechende Filter in einer der ersten Rotationsrichtung entgegengesetzten zweiten Rotationsrichtung von dem diskontinuierlichen Punkt um einen zweiten Betrag rotiert ist, auf die zweite Wellenlänge eingestellt ist.

6. Laserquellen-Einheit (13) nach einem der Ansprüche 1 bis 5,
wobei der doppelbrechende Filter eingerichtet ist, während einer Rotation in einer festen Richtung einen freien Spektralbereich umfassend die erste Wellenlänge und die zweite Wellenlänge eine Vielzahl von Malen zu wiederholen.

7. Laserquellen-Einheit (13) nach Anspruch 6, wobei der vorbestimmte Bereich, in welchem der doppelbrechende Filter hin und her rotiert wird kleiner ist als ein Bereich der Rotations-Verschiebung des doppelbrechenden Filters, der einem freien Spektralbereich entspricht.

8. Laserquellen-Einheit (13) nach einem der Ansprüche 1 bis 7, wobei die Oszillationswellenlänge des optischen Resonators sich in ein Sägezahn-Wellenmuster hinsichtlich der Rotations-Verschiebung des doppelbrechenden Filters ändert.

9. Fotoakustik-Bilderzeugungs-Vorrichtung umfassend:
eine Laserquellen-Einheit (13) nach einem der Ansprüche 1 bis 8;
ein Abtastmittel, das eingerichtet ist, ein fotoakustisches Signal abzutasten, das in einem Objekt generiert wird, wenn das Objekt mit dem gepulsten Laserstrahl mit einer Vielzahl von Wellenlängen bestrahlt wird, um fotoakustische Daten zu generieren, die den jeweiligen Wellenlängen entsprechen;
ein Phaseninformations-Extraktionsmittel, das eingerichtet ist, Phaseninformation zu erzeugen, die eine Stärkebeziehung zwischen relativen Signalintensitäten der fotoakustischen Daten, die den jeweiligen Wellenlängen entsprechen, angeben; und
ein Fotoakustik-Bildkonstruktions-Mittel, das eingerichtet ist, ein fotoakustisches Bild auf Basis der Phaseninformation zu erzeugen.

10. Fotoakustik-Bilderzeugungs-Vorrichtung (10) nach Anspruch 9, weiterhin umfassend ein Intensitätsinformations-Extraktionsmittel, das eingerichtet ist, eine Intensitätsinformation zu generieren, die eine Signalintensität auf der Basis der fotoakustischen Daten, die den jeweiligen Wellenlängen entsprechen, angibt,
wobei das Fotoakustik-Bildkonstruktions-Mittel eingerichtet ist, einen Gradationswert von jedem Pixel des Fotoakustikbilds auf Basis der Intensitätsinformation zu erzeugen und eine Displayfarbe von jedem Pixel auf Basis der Phaseninformation bestimmt.

11. Fotoakustik-Bilderzeugungs-Vorrichtung (10) nach Anspruch 10,
wobei die Vielzahl von Wellenlängen erste und zweite Wellenlängen umfasst,
wobei die Fotoakustik-Bilderzeugungs-Vorrichtung weiterhin umfasst:
ein Komplexzahl-Erzeugungsmittel, das eingerichtet ist, Komplexzahldaten zu generieren, in welchen eines von ersten Fotoakustikdaten, die einem Fotoakustiksignal entsprechen, das detektiert wird, wenn eine Bestrahlung mit einem gepulsten Laserstrahl mit der ersten Wellenlänge durchgeführt wird, und zweiten Fotoakustikdaten, die einem Fotoakustiksignal entsprechen, das detektiert wird, wenn eine Bestrahlung mit einem gepulsten Laserstrahl mit der zweiten Wellenlänge durchgeführt wird, als ein Realteil eingestellt ist und die anderen als ein Imaginärteil eingestellt ist; und
ein Fotoakustik-Bildrekonstruktions-Mittel, das eingerichtet ist, ein rekonstruiertes Bild von den Komplexzahldaten unter Verwendung einer Fouriertransformationsmethode zu erzeugen, und
wobei das Phaseninformations-Extraktionsmittel die Phaseninformation aus dem rekonstruierten Bild extrahiert und das Intensitätsinformations-Extraktionsmittel eingerichtet ist, die Intensitätsinformation von dem rekonstruierten Bild zu extrahieren.

12. Fotoakustik-Bilderzeugungs-Vorrichtung (10) nach einem der Ansprüche 9 bis 11,
wobei das Abtastmittel weiterhin eingerichtet ist, reflektierte Akustikwellen bezüglich zu dem Objekt transmittierten Akustikwellen abzutasten, um Reflexions-Akustikwellendaten zu erzeugen, und
wobei die Fotoakustik-Bilderzeugungs-Vorrichtung weiterhin ein Akustikwellen-Bilderzeugungsmittel umfasst, das eingerichtet ist, ein Akustikwellenbild auf Basis der Reflexions-Akustikwellendaten zu erzeugen.

13. Steuerverfahren für eine Laserquellen-Einheit (13), die in der Lage ist, gepulste Laserstrahlen mit einer Vielzahl von unterschiedlichen Wellenlängen zu emittieren, wobei das Steuerverfahren umfasst:
einen Schritt des Hin-und-her-Rotierens eines doppelbrechenden Filters (56) in einem vorbestimmten Bereich, der in einen optischen Resonator eingesetzt ist, umfassend ein Paar von einander gegenüberstehenden Spiegeln (53, 54), wobei ein Laserstab (51) dazwischen angeordnet ist, und der eine Oszillationswellenlänge des optischen Resonators in Verbindung mit einer Rotations-Verschiebung des doppelbrechenden Filters verändert; und
einen Schritt des Bestrahlens des Laserstabs mit Anregungslicht und nach dem Bestrahlen mit dem Anregungslicht, Einschalten eines Q-Switches (55), der in den optischen Resonator eingesetzt ist zu einem Zeitpunkt, wenn eine Rotationsverschiebungsposition des doppelbrechenden Filters auf eine Position eingestellt ist, die der zu emittierenden Wellenlänge des gepulsten Laserstrahls entspricht, um zu veranlassen, dass der gepulste Laserstrahl emittiert wird.

14. Fotoakustik-Bilderzeugungsverfahren umfassend:
ein Verfahren zur Steuerung einer Laserlichtquelleneinheit (13) nach Anspruch 13.

## Revendications

1. Unité de source laser (13), en mesure d'émettre des faisceaux laser à impulsions présentant différentes longueurs d'onde, l'unité de source laser comprenant :
un barreau laser (51) ;
une source de lumière d'excitation (52), laquelle irradie le barreau laser avec une lumière d'excitation ;
un résonateur optique, lequel inclut une paire de miroirs (53, 54) se faisant face, le barreau laser étant intercalé entre eux ;
un commutateur de facteur de qualité Q (55), lequel est introduit dans le résonateur optique ;
un filtre biréfringent (56), lequel est introduit dans le résonateur optique, et modifie une longueur d'onde d'oscillation du résonateur optique en association avec un déplacement de rotation dudit filtre biréfringent ;
un moyen d'entraînement (57), lequel est configuré pour entraîner en rotation réciproque le filtre biréfringent dans une plage prédéterminée incluant une position sur laquelle des caractéristiques de modification de la longueur d'onde d'oscillation pour le déplacement de rotation du filtre biréfringent sont réglées de manière à être un point discontinu, et
une unité de commande d'émission (59), laquelle est configurée pour irradier le barreau laser avec une lumière d'excitation à partir de la source de lumière d'excitation, et est configurée pour mettre sous tension le commutateur Q, sur une temporisation lorsqu'une position de déplacement de rotation du filtre biréfringent est réglée sur une position correspondant à la longueur d'onde du faisceau laser à impulsions à émettre, afin de provoquer l'émission du faisceau laser à impulsions.

2. Unité de source laser (13) selon la revendication 1, dans laquelle le moyen d'entraînement (57) inclut un corps rotatif (61), lequel est configuré pour se déplacer de manière circulaire, et un barreau (62) lequel relie le corps rotatif et le filtre biréfringent, et présente un mécanisme à manivelle (60), lequel est configuré pour convertir un mouvement circulaire du corps rotatif en un mouvement de rotation alternative du filtre biréfringent.

3. Unité de source laser (13) selon la revendication 2, dans laquelle le corps rotatif est configuré pour être entraîné en rotation à une vitesse fixe.

4. Unité de source laser (13) selon la revendication 1, dans laquelle le moyen d'entraînement présente un mécanisme convertissant un mouvement alternatif linéaire en un mouvement de rotation alternative dans une plage prédéterminée.

5. Unité de source laser (13) selon l'une quelconque des revendications 1 à 4,
dans laquelle la pluralité de longueurs d'onde inclut des première et seconde longueurs d'onde, et
dans laquelle la longueur d'onde d'oscillation du résonateur optique est réglée sur la première longueur d'onde sur une première position de déplacement de rotation sur laquelle le filtre biréfringent est entraîné en rotation dans une première direction de rotation à raison d'une première quantité à partir du point discontinu, et la longueur d'onde d'oscillation du résonateur optique est réglée sur la seconde longueur d'onde sur une seconde position de déplacement de rotation sur laquelle le filtre biréfringent est entraîné en rotation dans une seconde direction, opposée à la première direction, à raison d'une seconde quantité à partir du point discontinu.

6. Unité de source laser (13) selon l'une quelconque des revendications 1 à 5, dans laquelle le filtre biréfringent est configuré pour répéter une plage spectrale libre incluant la première longueur d'onde et la seconde longueur d'onde plusieurs fois durant une rotation dans une direction fixe.

7. Unité de source laser (13) selon la revendication 6, dans laquelle la plage prédéterminée sur laquelle le filtre biréfringent est entraîné en rotation alternative est plus petite qu'une plage du déplacement de rotation du filtre biréfringent correspondant à une plage spectrale libre.

8. Unité de source laser (13) selon l'une quelconque des revendications 1 à 7, dans laquelle la longueur d'onde d'oscillation du résonateur optique est modifiée sur un motif d'onde en dents de scie pour le déplacement de rotation du filtre biréfringent.

9. Appareil de génération d'image photoacoustique, comprenant :
une unité de source laser (13) selon l'une quelconque des revendications 1 à 8,
un moyen d'échantillonnage, lequel est configuré pour échantillonner un signal photoacoustique, généré dans les limites d'un objet lorsque l'objet est irradié avec le faisceau laser à impulsions présentant une pluralité de longueur d'ondes, afin de générer des parties de données photoacoustiques correspondant aux longueurs d'onde respectives ;
un moyen d'extraction d'informations de phase, lequel est configuré pour générer des informations de phase indiquant une relation de grandeur entre des intensités de signal relatives des parties de données photoacoustiques correspondant aux longueurs d'onde respectives, et
un moyen de construction d'image photoacoustique, lequel est configuré pour générer une image photoacoustique sur la base des informations de phase.

10. Appareil de génération d'image photoacoustique (10) selon la revendication 9, comprenant en outre un moyen d'extraction d'informations d'intensité, lequel est configuré pour générer des informations d'intensité indiquant une intensité de signal sur la base des parties de données photoacoustiques correspondant aux longueurs d'onde respectives, et
dans lequel le moyen de de construction d'image photoacoustique est configuré pour générer une valeur de gradation de chaque pixel de l'image photoacoustique sur la base des informations d'intensité, et détermine une couleur d'affichage de chaque pixel sur la base des informations de phase.

11. Appareil de génération d'image photoacoustique (10) selon la revendication 10,
dans lequel la pluralité de longueurs d'onde inclut des première et seconde longueurs d'onde,
dans lequel l'appareil de génération d'image photoacoustique comprend en outre :
un moyen de création de nombre complexe, lequel est configuré pour générer des données de nombre complexe où l'une quelconque des données parmi des premières données photoacoustiques correspondant à un signal photoacoustique, détectée lorsqu'est réalisée une irradiation avec un faisceau laser à impulsions présentant la première longueur d'onde, et des secondes données photoacoustiques correspondant à un signal photoacoustique, détectée lorsqu'est réalisée une irradiation avec un faisceau laser à impulsions présentant la seconde longueur d'onde, est réglée sur une partie réelle, et l'autre est réglée sur une partie imaginaire, et
un moyen de reconstruction d'image photoacoustique, lequel est configuré pour générer une image reconstruite à partir des données de nombre complexe à l'aide d'une méthode de transformée de Fourier, et
dans lequel le moyen d'extraction d'informations de phase extrait les informations de phase de l'image reconstruite, et le moyen d'extraction d'informations d'intensité est configuré pour extraire les informations d'intensité de l'image reconstruite.

12. Appareil de génération d'image photoacoustique (10) selon l'une quelconque des revendications 9 à 11,
dans lequel le moyen d'échantillonnage est en outre configuré pour échantillonner des ondes acoustiques réfléchies par rapport à des ondes acoustiques transmises à l'objet pour générer des données d'onde acoustiques réfléchies, et
dans lequel l'appareil de génération d'image photoacoustique comprend en outre un moyen de génération d'image d'onde acoustique, lequel est configuré pour générer une image d'onde acoustique sur la base des données d'ondes acoustiques réfléchies.

13. Procédé de commande d'une unité de source laser (13), en mesure d'émettre des faisceaux laser à impulsions présentant une pluralité de longueurs d'onde différentes, le procédé de commande comprenant :
une étape pour entraîner en rotation alternative, dans une plage prédéterminée, un filtre biréfringent (56), lequel est introduit dans un résonateur optique incluant une paire de miroirs (53, 54) se faisant face, un barreau laser (51) étant intercalé entre eux, et modifie une longueur d'onde d'oscillation du résonateur optique en association avec un déplacement de rotation dudit filtre biréfringent, et
une étape pour irradier le barreau laser avec une lumière d'excitation, et après irradiation avec la lumière d'excitation, pour mettre sous tension un commutateur Q (55) introduit dans le résonateur optique, sur une temporisation lorsqu'une position de déplacement de rotation du filtre biréfringent est réglée sur une position correspondant à la longueur d'onde du faisceau laser à impulsions à émettre, afin de provoquer l'émission du faisceau laser à impulsions.

14. Procédé de génération d'image photoacoustique, comprenant :
un procédé de commande d'une unité de source de lumière laser (13) selon la revendication 13.
